# EUROPÄISCHE PATENTSCHRIFT

(11) **EP 4 178 938 B1**
(45) Veröffentlichungstag und Bekanntmachung des Hinweises auf die Patenterteilung: **04.09.2024**
(21) Anmeldenummer: 21749112.5
(22) Anmeldetag: 12.07.2021
(51) Int. Cl.: C07C 41/42, C07C 41/56, C07C 43/30, C07C 45/42

(54) **VERFAHREN ZUR HERSTELLUNG VON POLYOXYMETHYLENDIMETHYLETHERN**
METHOD FOR PRODUCING POLYOXYMETHYLENE DIMETHYL ETHERS
PROCÉDÉ DE PRODUCTION D'ÉTHERS DIMÉTHYLIQUES DE POLYOXYMÉTHYLÈNE

(30) Priorität: 13.07.2020 DE 102020118386
(43) Veröffentlichungstag der Anmeldung: 17.05.2023
(73) Patentinhaber: Fraunhofer-Gesellschaft zur Förderung der angewandten Forschung e.V., 80686 München (DE)
(72) Erfinder: MANTEI, Franz, 79110 Freiburg (DE); OUDA, Mohamed, 79110 Freiburg (DE); SCHAADT, Achim, 79110 Freiburg (DE)
(74) Vertreter: Maiwald GmbH
(86) Internationale Anmeldenummer: PCT/EP2021/069282
(87) Internationale Veröffentlichungsnummer: WO 2022/013132

(56) Entgegenhaltungen:
- DE-A1- 102016 222 657

## Beschreibung

Die vorliegende Erfindung betrifft ein Verfahren zur Herstellung von Polyoxymethylendimethylethern.

Synthetische Energieträger, welche nicht auf Basis von Rohöl oder Erdgas hergestellt werden, können die Abhängigkeit von fossilen Energiequellen sowie die aus deren Verwendung entstehende Umweltbelastung verringern. Ein Beispiel für einen solchen Energieträger sind Polyoxymethylendimethylether (OME). Polyoxymethylendimethylether (OME) können aus Kohlenstoffdioxid und Wasser hergestellt werden und weisen das Potential auf, soweit regenerative Energieträger für deren Herstellung eingesetzt werden, bei der Umsetzung als Kraftstoff den Kohlenstoffdioxidkreislauf zu schließen.

Darüber hinaus bietet die Nutzung der Polyoxymethylendimethylether als Energieträger weitere Vorteile. Polyoxymethylendimethylether weisen keine Kohlenstoff-Kohlenstoff-Bindungen auf und besitzen außerdem einen hohen Anteil an Sauerstoff. Polyoxymethylendimethylether verbrennen rußfrei und schonen damit sowohl den Verbrennungsmotor und nachgeschaltete Filterelemente als auch die Umwelt. Weiterhin ermöglicht die rußfreie Verbrennung eine Verringerung der Stickoxidemissionen. Polyoxymethylendimethylether mit drei bis fünf Oxymethylen-Einheiten (OME₃₋₅) sind dabei aufgrund ihrer dieselähnlichen Eigenschaften von besonderem Interesse.

Bei der Synthese von Polyoxymethylendimethylethern werden als Reaktanten üblicherweise eine Formaldehydquelle (z.B. Formaldehyd, Trioxan oder Paraformaldehyd) und eine Verbindung für die Methylverkappung (engl.: "*methyl capping*") wie Methanol, Methylal oder Dimethylether verwendet. Enthält das Reaktantengemisch Methanol und Wasser, reagieren diese mit Formaldehyd zu Polyoxymethylenglykolen (MGₙ; HO-(CH₂O)ₙ-H) und Polyoxymethylen-Halbacetalen (HFₙ; HO-(CH₂O)ₙ-CH₃) gemäß den nachfolgenden Reaktionsgleichungen 1-4. Diese Reaktionen benötigen nicht die Anwesenheit eines Katalysators und erreichen sehr schnell das chemische Gleichgewicht. Außerdem liegt das chemische Gleichgewicht ganz überwiegend auf der Produktseite, so dass im Produktgemisch im Wesentlichen kein monomeres Formaldehyd (CH₂O) vorliegt. Für die Bildung von Methylal (H₃C-O-(CH₂O)₁-CH₃; OME₁) durch die Acetalisierungsreaktion zwischen Methanol und HO-CH₂O-CH₃ (HF₁) gemäß der nachfolgenden Reaktionsgleichung 5 wird die Anwesenheit eines sauren Katalysators benötigt. Kettenwachstum durch den Einbau weiterer CH₂O-Einheiten erfolgt gemäß der nachfolgenden Reaktionsgleichung 7. Weitere Acetalisierungsreaktionen zwischen Methanol und den Polyoxymethylen-Halbacetalen HFₙ erfolgen gemäß der nachfolgenden Reaktionsgleichung 6.

Mögliche Nebenreaktionen unter Bildung von Trioxan ((CH₂CO₃) und Ameisensäuremethylester (HC(O)OCH₃) zeigen die nachfolgenden Reaktionsgleichungen 8 und 9.

CH₂O + H₃C-OH ⇆ HO-(CH₂O)₁-CH₃ 1

CH₂O + HO-(CH₂O)ₙ₋₁-CH₃ ⇆ HO-(CH₂O)ₙ-CH₃, n ≥ 2 2

CH₂O + H₂O ⇆ HO-(CH₂O)₁-H 3

CH₂O + HO-(CH₂O)ₙ₋₁-H ⇆ HO-(CH₂O)ₙ-H, n ≥ 2 4

H₃C-OH + HO-(CH₂O)₁-CH₃ ⇆^{H+} H₃C-O-(CH₂O)₁-CH₃ + H₂O 5

H₃C-OH + HO-(CH₂O)ₙ-CH₃ ⇆^{H+} H₃C-O-(CH₂O)ₙ-CH₃ + H₂O 6

CH₂O + H₃C-O-(CH₂0)ₙ₋₁-CH₃ ⇆^{H+} H₃C-O-(CH₂O)ₙ-CH₃, n ≥ 2 7

3 CH₂O ⇆^{H+} (CH₂O)₃ 8

2 CH₂O → HCOOCH₃ 9

Eine Übersicht bekannter Herstellungsverfahren für Polyoxymethylendimethylether H₃C-O-(CH₂O)ₙ-CH₃ mit n≥2 (OME_{≥2}), insbesondere n = 3-5 (OME₃₋₅), findet sich z.B. in der Publikation von M. Ouda et al., React. Chem. Eng., 2017, 2, S. 50-59. Dabei wird zwischen wasserfreien und wässrigen Syntheserouten unterschieden. Wasserfreie Syntheserouten weisen eine verringerte Bildung von Nebenprodukten auf, jedoch ist die Bereitstellung der Reaktanten energieintensiv. Die Bereitstellung der Reaktanten für wässrige Syntheserouten ist weniger energieintensiv, jedoch sind im Reaktionsprodukt weitere Nebenprodukte und Wasser enthalten.

Beispielsweise kann von einem Reaktantengemisch ausgegangen werden, das Methylal und Trioxan enthält. Bei dieser Synthesevariante ist vorteilhaft, dass sie die Polyoxymethylendimethylether in hoher Ausbeute herstellt und im Wesentlichen in Abwesenheit von Wasser durchführbar ist, was die Anzahl der Nebenprodukte verringert. Nachteilig ist, dass die Herstellung von wasserfreiem Trioxan sehr energieintensiv und komplex ist, was die Energieeffizienz und die wirtschaftliche Machbarkeit des Prozesses negativ beeinflusst.

US 2007/260094 A1 beschreibt ein Verfahren zur Herstellung von Polyoxymethylendimethylethern, bei dem Methylal und Trioxan in einen Reaktor eingespeist werden und in Gegenwart eines sauren Katalysators umgesetzt werden, wobei die in das Reaktionsgemisch eingebrachte Wassermenge weniger als 1 Gew% beträgt.

Eine im Wesentlichen wasserfreie Synthese von Polyoxymethylendialkylethern wird auch durch die Verwendung von Trioxan und einem Dialkylether (wie z.B. Dimethylether DME) als Reaktanten ermöglicht.

DE 10 2005 027690 A1 beschreibt ein Verfahren zur Herstellung von Polyoxymethylendialkylethern, bei dem ein Dialkylether (Dimethylether, Methylethylether oder Diethylether) und Trioxan in einen Reaktor eingespeist werden und in Gegenwart eines sauren Katalysators umgesetzt werden, wobei die durch den Dialkylether, Trioxan und/oder den Katalysator in das Reaktionsgemisch eingebrachte Wassermenge weniger als 1 Gew% beträgt. P. Haltenort et al., Catalysis Communications, 2018, 109, 80, beschreiben die Synthese von Polyoxymethylendimethylethern aus Dimethylether (DME) und Trioxan unter Verwendung eines Zeolithen als sauren Katalysator. Der maximale DME-Umsatz betrug 13,9 Gew% und die maximale Ausbeute an OME₃₋₅, bezogen auf die eingesetzten Reaktanten, betrug 8,2 Gew%. Untersuchungen haben gezeigt, dass die von Dimethylether und Trioxan ausgehende Synthesevariante zu relativ hohen Reaktorverweilzeiten führt.

Weiterhin ist bekannt, den für die Polyoxymethylendimethylether-Synthese verwendeten Formaldehyd über eine katalytische Dehydrierung von Methanol herzustellen, siehe z.B. M. Ouda, F. Mantei et al., React. Chem. Eng., 2018, 129, 11164. Dieser Ansatz ermöglicht den Einsatz eines wasserfreien Reaktantengemisches, so dass lediglich das während der Synthese entstehende Wasser im Produktgemisch enthalten ist. Allerdings handelt es sich bei der katalytischen Dehydrierung von Methanol um eine komplexe chemische Reaktion, deren Technologie-Reifegrad noch relativ gering ist.

Bekannt ist auch die Verwendung eines wässrigen formaldehyd- und methanolhaltigen Reaktantengemisches.

DE 10 2016 222657 A1 beschreibt ein Verfahren zur Herstellung von Polyoxymethylendimethylethern, das folgende Schritte umfasst:
(i) Einspeisung von Formaldehyd, Methanol und Wasser in einen Reaktor R und Umsetzung zu einem Reaktionsgemisch enthaltend Formaldehyd, Wasser, Methylenglykol, Polyoxymethylenglykole, Methanol, Hemiformale, Methylal und Polyoxymethylendimethylether;
(ii) Einspeisung des Reaktionsgemischs in eine Reaktivdestillationskolonne K1 und Auftrennung in eine Leichtsiederfraktion F1 enthaltend Formaldehyd, Wasser, Methylenglykol, Polyoxymethylenglykole, Methanol, Hemiformale, Methylal und Polyoxymethylendimethylether mit 2 bis 3 Oxymethylen-Einheiten (OME₂₋₃), und eine Schwersiederfraktion F2 enthaltend Polyoxymethylendimethylether mit mehr als zwei Oxymethylen-Einheiten (OME_{≥3}).

Eine wässrige Formaldehydlösung weist im Vergleich zu einer wasserfreien Formaldehydquelle wie Trioxan einen geringeren Energieaufwand bei ihrer Herstellung auf. Bei der Verwendung von Reaktanten in wässriger Phase stellt sich allerdings die Herausforderung, das Wasser möglichst effizient aus dem Produktgemisch zu entfernen. Zudem führt die Anwesenheit von Wasser zur Bildung weiterer Nebenprodukte, was die Produktausbeute verringert.

Nachteilig bei der Verwendung von Methanol als Reaktant ist, dass durch die Acetalisierungsreaktion zwischen Methanol und den Halbacetalen zusätzliches Wasser als Nebenprodukt entsteht (siehe die obigen Reaktionsgleichungen 5 und 6) und dieses Reaktionswasser ebenfalls aus dem Verfahren zu entfernen ist. Aufgrund des komplexen Destillationsverhaltens des Produktgemisches kann das Wasser nicht über eine Standarddestillation abgetrennt werden, ohne gleichzeitig auch einen Teil des Formaldehyds abzutrennen. Alternativ zur destillativen Abtrennung wurden daher andere Methoden zur Wasserabtrennung wie z.B. Adsorption, Membran-basierte Trennverfahren oder Extraktion untersucht. Diese Methoden weisen jedoch weitere Herausforderungen für den Langzeitbetrieb auf. Diese alternativen Wasserabtrennmethoden werden beispielsweise in den folgenden Publikationen beschrieben:
- N. Schmitz et al., Industrial & Engineering Chemistry Research, 2017, 56, 11519;
- N. Schmitz et al., Journal of Membrane Science, 2018, 564, 806;
- L. Wang et al., J. Chem. Eng. Data, 2018, 63, 3074;
- M. Shi et al., Can. J. Chem. Eng., 2018, 96, 968;
- D. Oestreich et al., Fuel, 2018, 214, 39;
- X. Li et al., J. Chem. Eng. Data, 2019, 64, 5548.

Methylal findet Verwendung als Lösungsmittel und in der Produktion von Parfüm, Harzen oder Schutzanstrichen. Es wird außerdem als Kraftstoffadditiv und als synthetischer Kraftstoff erprobt. Die Herstellung von möglichst reinem Methylal wird beispielsweise in WO 2012/062822 A1 beschrieben. In diesem Verfahren reagieren Formaldehyd und Methanol zu einem Produktgemisch, das Methylal und Wasser sowie nicht umgesetztes Methanol und Formaldehyd enthält. In einer Reaktivdestillationseinheit wird das Produktgemisch in drei Fraktionen aufgetrennt. Die Fraktion, die die Reaktivdestillationseinheit als Kopfstrom verlässt, ist reich an Methylal. Die Herstellung von Polyoxymethylendimethylethern wird nicht beschrieben.

Eine Aufgabe der vorliegenden Erfindung ist die Herstellung von Polyoxymethylendimethylethern über ein möglichst effizientes und gut skalierbares Verfahren.

Gelöst wird die Aufgabe durch die beiden nachfolgend beschriebenen alternativen erfindungsgemäßen Verfahren ("erste unabhängige Ausführungsform" und "zweite unabhängige Ausführungsform").

Gemäß einer ersten unabhängigen Ausführungsform der vorliegenden Erfindung wird die Aufgabe gelöst durch ein Verfahren zur Herstellung von Polyoxymethylendimethylethern, das folgende Schritte umfasst:
- Mischen eines Stroms S_{FA}, der eine wasserhaltige Formaldehydquelle enthält, mit einem Strom S_{OME1}, der Methylal enthält, unter Erhalt eines Reaktantengemisches M_{Reaktant},
- Umsetzen des Reaktantengemisches M_{Reaktant} in einem Reaktor R1 unter Erhalt eines Produktgemisches M_{R1}, das Polyoxymethylendimethylether der Formeln H₃C-O-(CH₂O)₂-CH₃ (OME₂), H₃C-O-(CH₂O)₃₋₅-CH₃ (OME₃₋₅) und H₃C-O-(CH₂-O)ₙ-CH₃ mit n≥6 (OME_{≥6}) sowie Formaldehyd, Methylal (OME₁), Methanol und Wasser enthält,
- Einleiten des Produktgemisches M_{R1} in eine Destillationseinheit D1 und destillatives Auftrennen des Produktgemisches M_{R1} in eine erste Fraktion, die Methylal (OME₁), H₃C-O-(CH₂O)₂-CH₃ (OME₂), Formaldehyd, Methanol und Wasser enthält und die Destillationseinheit D1 als Kopfstrom KS_{D1} verlässt, sowie eine zweite Fraktion, die die Polyoxymethylendimethylether der Formeln H₃C-O-(CH₂O)₃₋₅-CH₃ (OME₃₋₅) und H₃C-O-(CH₂O)ₙ-CH₃ mit n≥6 (OME₂₆) enthält und die Destillationseinheit D1 als Sumpfstrom SS_{D1} verlässt,
- Mischen des Kopfstroms KS_{D1} mit einem methanolhaltigen Strom S_{MeOH} unter Erhalt eines Gemisches M1,
- Umsetzen des Gemisches M1 in mindestens einer Reaktionszone RZ einer Reaktivdestillationseinheit RD2 in Gegenwart eines sauren Katalysators, wobei H₃C-O-(CH₂O)₂-CH₃ (OME₂) zu Methylal (OME₁) und Formaldehyd reagiert und Formaldehyd und Methanol zu Methylal (OME₁) reagieren; und destillatives Auftrennen in eine erste Fraktion, die Methylal (OME₁) enthält und die Reaktivdestillationseinheit RD2 als Kopfstrom KS_{RD2} verlässt, und eine zweite Fraktion, die Wasser enthält und die Reaktivdestillationseinheit RD2 als Sumpfstrom SS_{RD2} verlässt,
- Einleiten des Sumpfstroms SS_{D1} in eine Destillationseinheit D3 und destillatives Auftrennen der im Sumpfstrom SS_{D1} vorliegenden Polyoxymethylendimethylether der Formeln H₃C-O-(CH₂O)₃₋₅-CH₃ (OME₃₋₅) und H₃C-O-(CH₂O)ₙ-CH₃ mit n≥6 (OME₂₆) in eine Fraktion, die die Destillationseinheit D3 als Kopfstrom KS_{D3} verlässt, und eine Fraktion, die die Destillationseinheit als Sumpfstrom SS_{D3} verlässt.

Alternativ wird die Aufgabe gemäß einer zweiten unabhängigen Ausführungsform gelöst durch ein Verfahren zur Herstellung von Polyoxymethylendimethylethern, das folgende Schritte umfasst:
- Mischen eines Stroms S_{FA}, der eine wasserhaltige Formaldehydquelle enthält, mit einem Strom S_{OME1}, der Methylal enthält, unter Erhalt eines Reaktantengemisches M_{Reaktant},
- Umsetzen des Reaktantengemisches M_{Reaktant} in einem Reaktor R1 unter Erhalt eines Produktgemisches M_{R1}, das Polyoxymethylendimethylether der Formeln H₃C-O-(CH₂O)₂-CH₃ (OME₂), H₃C-O-(CH₂O)₃₋₅-CH₃ (OME₃₋₅) und H₃C-O-(CH₂O)ₙ-CH₃ mit n≥6 (OME₂₆) sowie Formaldehyd, Methylal (OME₁), Methanol und Wasser enthält,
- Mischen des Produktgemisches M_{R1} mit einem methanolhaltigen Strom S_{MeOH} unter Erhalt eines Gemisches M1,
- Umsetzen des Gemisches M1 in mindestens einer Reaktionszone RZ einer Reaktivdestillationseinheit RD1 in Gegenwart eines sauren Katalysators, wobei H₃C-O-(CH₂O)₂-CH₃ (OME₂) zu Methylal (OME₁) und Formaldehyd reagiert und Formaldehyd und Methanol zu Methylal (OME₁) reagieren; und destillatives Auftrennen in eine erste Fraktion, die Wasser, Methanol, Formaldehyd, Methylal (OME₁) und H₃C-O-(CH₂O)₂-CH₃ (OME₂) enthält und die Reaktivdestillationseinheit RD1 als Kopfstrom KS_{RD1} verlässt, und eine zweite Fraktion, die die Polyoxymethylendimethylether der Formeln H₃C-O-(CH₂O)₃₋₅-CH₃ (OME₃₋₅) und H₃C-O-(CH₂O)ₙ-CH₃ mit n≥6 (OME₂₆) enthält und die Reaktivdestillationseinheit RD1 als Sumpfstrom SS_{RD1} verlässt,
- Einleiten des Kopfstroms KS_{RD1} in eine Destillationseinheit D2 und destillatives Auftrennen in eine erste Fraktion, die Methylal enthält und die Destillationseinheit D2 als Kopfstrom KS_{D2} verlässt, und eine zweite Fraktion, die Wasser enthält und die Destillationseinheit D2 als Sumpfstrom SS_{D2} verlässt,
- Einleiten des Sumpfstroms SS_{RD1} in eine Destillationseinheit D3 und destillatives Auftrennen der im Sumpfstrom SS_{RD1} vorliegenden Polyoxymethylendimethylether der Formeln H₃C-O-(CH₂O)₃₋₅-CH₃ (OME₃₋₅) und H₃C-O-(CH₂O)ₙ-CH₃ mit n≥6 (OME₂₆) in eine Fraktion, die die Destillationseinheit D3 als Kopfstrom KS_{D3} verlässt, und eine Fraktion, die die Destillationseinheit als Sumpfstrom SS_{D3} verlässt.

Wie nachfolgend noch eingehender beschrieben wird, ermöglichen die erfindungsgemäßen Verfahren eine effektive destillative Abtrennung des Wassers und adressieren damit eine der Hauptherausforderungen der Herstellungsprozesse von Polyoxymethylenethern. Das im Gemisch M1 vorliegende Formaldehyd und das während der Reaktionen von OME₂ zu Methylal und Formaldehyd entstehende Formaldehyd wird durch das im Kopfstrom KS_{D1} enthaltene Methanol (erste unabhängige Ausführungsform der Erfindung) bzw. das im Produktgemisch M_{R1} enthaltene Methanol (zweite unabhängige Ausführungsform der Erfindung) und das über den methanolhaltigen Strom S_{MeOH} extern definiert hinzugegebene Methanol chemisch in Form von Methylal gebunden. Durch den externen Methanolstrom S_{MeOH} kann damit der Formaldehydanteil im Sumpfstrom SS_{RD2} (erste unabhängige Ausführungsform der Erfindung) bzw. im Kopfstrom KS_{RD1} und im Sumpfstrom SS_{D2} (zweite unabhängige Ausführungsform der Erfindung) gezielt eingestellt (z.B. im Wesentlichen vollständig entfernt) werden. Die Verwendung zusätzlicher Wasserabtrenneinheiten ist nicht erforderlich.

Die in den beiden unabhängigen Ausführungsformen der vorliegenden Erfindung verwendete wasserhaltige Formaldehydquelle ist bevorzugt eine wässrige Formaldehydlösung, insbesondere eine konzentrierte wässrige Formaldehydlösung mit einem Formaldehydgehalt von mindestens 70 Gew%, bevorzugter mindestens 80 Gew%, noch bevorzugter mindestens 90 Gew%. Solche wässrigen Formaldehydlösungen sind kommerziell erhältlich oder lassen sich über bekannte Verfahren herstellen, z.B. aus einer wässrigen formaldehydhaltigen Ausgangslösung, die eine Konzentratoreinheit (z.B. einen oder mehrere Dünnschichtverdampfer) durchläuft und so in eine konzentrierte wässrige Formaldehydlösung überführt wird. Die Herstellung einer konzentrierten wässrigen Formaldehydlösung wird beispielsweise in WO 03/040075 A2, EP 1 688 168 A1, DE 103 09 289 A1 oder DE 103 09 286 A1 beschrieben.

Der formaldehyd- und wasserhaltige Strom S_{FA} weist somit bevorzugt einen Gehalt an Formaldehyd von mindestens 70 Gew%, bevorzugter mindestens 80 Gew%, noch bevorzugter mindestens 90 Gew%, beispielsweise im Bereich von 70-97 Gew%, bevorzugter 80-95 Gew% oder 90-95 Gew% auf.

Wie dem Fachmann bekannt ist, liegt in einer wässrigen Formaldehydlösung der monomere Formaldehyd CH₂O neben seinem monomeren Hydrat Methylenglykol (HO-(CH₂O)₁-H) und seinen oligomeren Hydraten, auch als Polyoxymethylenglykole (HO-(CH₂O)ₙ-H mit n≥2) bezeichnet, vor. Der Formaldehydgehalt der wässrigen Formaldehydlösung bezieht sich auf die Gesamtmenge an monomerem Formaldehyd CH₂O, monomerem Formaldehydhydrat (d.h. Methylenglykol (HO-(CH₂O)₁-H) und oligomeren Formaldehydhydraten (d.h. Polyoxymethylenglykolen (HO-(CH₂O)ₙ-H mit n≥2).

Sowohl in der ersten als auch der zweiten Ausführungsform des erfindungsgemäßen Verfahrens wird der formaldehyd- und wasserhaltige Strom S_{FA} mit einem Strom S_{OME1}, der Methylal (H₃C-O-(CH₂O)₁-CH₃, auch als Dimethoxymethan oder OME₁ bezeichnet) enthält, unter Erhalt eines Reaktantengemisches M_{Reaktant} gemischt. Wie nachfolgend noch eingehender beschrieben wird, handelt es sich bei dem methylalhaltigen Strom S_{OME1} bevorzugt um den aus der Reaktivdestillationseinheit RD2 abgezogenen Kopfstrom KS_{RD2} (erste Ausführungsform der Erfindung) bzw. den aus der Destillationseinheit D2 abgezogenen Kopfstrom KS_{D2} (zweite Ausführungsform der Erfindung), der für das Mischen mit der wasserhaltigen Formaldehydquelle S_{FA} zurückgeführt wurde. Zum Anfahren des erfindungsgemäßen Prozesses kann eine andere Methylalquelle verwendet werden. Bevorzugt weist der methylalhaltige Strom S_{OME1} einen Gehalt an Methylal von mindestens 70 Gew%, bevorzugter mindestens 90 Gew% auf. Optional kann der methylalhaltige Strom S_{OME1} weitere Komponenten wie z.B. Methanol enthalten. Der Gehalt an Methanol in S_{OME1} ist jedoch bevorzugt < 10 Gew%.

Das Mischen des formaldehyd- und wasserhaltigen Stroms S_{FA} mit dem methylalhaltigen Strom S_{OME1} erfolgt bevorzugt außerhalb des Reaktors R1 und das Reaktantengemisch M_{Reaktant} wird anschließend in den Reaktor R1 eingeleitet. Alternativ ist es aber auch möglich, dass die Ströme S_{FA} und S_{OME1} erst im Reaktor R1 miteinander gemischt werden.

Neben Formaldehyd, Methylal und Wasser kann das Reaktantengemisch M_{Reaktant} optional noch weitere Komponenten wie z.B. Methanol oder mehrere längerkettige Polyoxymethylendimethylether der allgemeinen Formel H₃C-O-(CH₂O)ₙ-CH₃ mit n≥6 (OME₂₆) enthalten. Die längerkettigen Polyoxymethylendimethylether wurden beispielsweise als Sumpfstrom SS_{D3} aus der Destillationseinheit D3 abgezogen und zurückgeführt.

Das molare Verhältnis von Methylal zu Formaldehyd in dem Reaktantengemisch M_{Reaktant} liegt beispielsweise im Bereich von 0,3 bis 2,0, bevorzugter 0,5 bis 1,5. Es können jedoch auch niedrigere oder höhere Werte für das molare Methylal/Formaldehyd-Verhältnis gewählt werden. Das bevorzugte molare Verhältnis hängt von dem Formaldehydgehalt des formaldehyd- und wasserhaltigen Stroms S_{FA} und dem Methylalgehalt des methylalhaltigen Strom S_{OME1} ab.

Sowohl in der ersten als auch der zweiten Ausführungsform des erfindungsgemäßen Verfahrens erfolgt die Umsetzung des Reaktantengemisches M_{Reaktant} in einem Reaktor R1 unter Erhalt eines Produktgemisches M_{R1}, das Polyoxymethylendimethylether der Formeln H₃C-O-(CH₂O)₂-CH₃ (OME₂), H₃C-O-(CH₂O)₃₋₅-CH₃ (OME₃₋₅) und H₃C-O-(CH₂O)ₙ-CH₃ mit n≥6 (OME₂₆) sowie Formaldehyd, Methylal (OME₁), Methanol und Wasser enthält. Geeignete Bedingungen für die Bildung von Polyoxymethylendimethylethern OME_{≥2} aus den Reaktanten Formaldehyd und Methylal (OME₁) sind dem Fachmann bekannt. Bevorzugt erfolgt die Umsetzung in Anwesenheit eines sauren Katalysators. Dabei können Feststoff-Katalysatoren oder auch flüssige Säuren verwendet werden. Beispielhaft können folgende Katalysatoren genannt werden: Ein lonenaustauscherharz, das saure Gruppen aufweist (d.h. ein Kationenaustauscherharz), ein Zeolith, ein Aluminosilicat, ein Aluminiumoxid, ein Übergangsmetalloxid (das optional auf einem Trägermaterial vorliegt), ein Graphenoxid, eine Mineralsäure (z.B. Schwefelsäure), eine organische Säure (z.B. eine Sulfonsäure), eine saure ionische Flüssigkeit, ein Oxoniumsalz (z.B. ein Trimethyloxoniumsalz). Der Reaktor R1 wird beispielsweise bei einem Druck von 1-10 bar und einer Temperatur von 50-120 °C betrieben. Der Reaktor R1 ist beispielsweise ein Festbettreaktor. Im Rahmen der vorliegenden Erfindung können jedoch auch andere Reaktortypen für die Umsetzung des Reaktantengemisches M_{Reaktant} verwendet werden. Neben OME₂, OME₃₋₅, OME_{≥6}, Formaldehyd, OME₁, Methanol und Wasser kann das Produktgemisch M_{R1} optional noch weitere Komponenten enthalten, beispielsweise Halbacetale der Formel HO-(CH₂O)ₙ-CH₃ mit n≥1, Glykole der Formel HO-(CH₂O)ₙ-H mit n≥1, Trioxan und/oder Ameisensäuremethylester.

Gemäß der ersten unabhängigen Ausführungsform des erfindungsgemäßen Verfahrens erfolgt in einer Destillationseinheit D1 ein destillatives Auftrennen des Produktgemisches M_{R1} in eine erste Fraktion, die Methylal (OME₁), H₃C-O-(CH₂O)₂-CH₃ (OME₂), Formaldehyd, Methanol und Wasser enthält und die Destillationseinheit D1 als Kopfstrom KS_{D1} verlässt, sowie eine zweite Fraktion, die die Polyoxymethylendimethylether der Formeln H₃C-O-(CH₂O)₃₋₅-CH₃ (OME₃₋₅) und H₃C-O-(CH₂O)ₙ-CH₃ mit n≥6 (OME₂₆) enthält und die Destillationseinheit D1 als Sumpfstrom SS_{D1} verlässt. Weitere Komponenten, die optional in dem Kopfstrom KS_{D1} vorliegen können, sind z.B. Halbacetale der Formel HO-(CH₂O)ₙ-CH₃ n≥2, Glykole der Formel HO-(CH₂O)ₙ-H mit n≥1, OME₃, Trioxan und/oder Ameisensäuremethylester. Bevorzugt enthält der Kopfstrom KS_{D1} kein OME_{≥4}, noch bevorzugter kein OME_{≥3}. Bevorzugt ist die Destillationseinheit D1 katalysatorfrei (insbesondere frei von sauren Katalysatoren). Bevorzugt ist die Destillationseinheit D1 keine Reaktivdestillationseinheit.

Die Destillationseinheit D1 wird also hinsichtlich der destillativen Auftrennung der Polyoxymethylendimethylether OME₂, OME₃₋₅ und OME_{≥6} so ausgelegt, dass OME₁₋₂ im Kopfstrom und OME_{≥3} im Sumpfstrom aus der Destillationseinheit D1 abgezogen werden. Hierfür geeignete Bedingungen kann der Fachmann unter Berücksichtigung des allgemeinen Fachwissens ermitteln. Die Destillationseinheit D1 ist beispielsweise eine Destillationskolonne. Die Destillationseinheit enthält üblicherweise Einbauten für die destillative Trennung, insbesondere Böden, Füllkörper oder strukturierte Packungen, wie sie dem Fachmann allgemein bekannt sind. Beispielsweise wird die Destillationseinheit D1 bei einem Druck von 1-15 bar und einer Temperatur von 60-250 °C betrieben. Um gegebenenfalls die Rückreaktion längerkettiger Halbacetale und Glykole zu Formaldehyd, Methanol, Wasser, HO-(CH₂O)₁-CH₃ (HF₁) und HO-(CH₂O)₁-H (MG₁) gemäß den oben genannten Reaktionsgleichungen 1-4 zu unterstützen, kann es vorteilhaft sein, eine relativ lange Aufenthaltszeit in der Destillationseinheit D1 zu wählen. Maßnahmen, mit denen sich die Aufenthaltszeit gegebenenfalls verlängern lässt, sind dem Fachmann bekannt. Beispielhaft kann in diesem Zusammenhang auf die in Absatz [0068] der DE 10 2016 222 657 A1 beschriebenen Maßnahmen verwiesen werden. Beispielsweise enthält die Destillationseinheit D1 Anstaupackungen (wie sie z.B. in EP 1 074 296 A1 beschrieben werden) oder Verweilzeitböden (z.B. Thormann^{®}-Böden).

Der in der ersten unabhängigen Ausführungsform des erfindungsgemäßen Verfahrens aus der Destillationseinheit D1 abgezogene Kopfstrom KS_{D1} wird mit einem methanolhaltigen Strom S_{MeOH} unter Erhalt eines Gemisches M1 gemischt. Optional kann der methanolhaltige Strom S_{MeOH} noch weitere Komponenten (z.B. Formaldehyd, Wasser, OME₁, OME_{≥2} oder Trioxan) enthalten. Bevorzugt ist jedoch, dass der methanolhaltige Strom S_{MeOH} mindestens 80 Gew% Methanol, bevorzugter mindestens 90 Gew% Methanol enthält.

In der ersten unabhängigen Ausführungsform des erfindungsgemäßen Verfahrens erfolgen ein Umsetzen des Gemisches M1 in mindestens einer Reaktionszone RZ einer Reaktivdestillationseinheit RD2 in Gegenwart eines Katalysators, wobei H₃C-O-(CH₂O)₂-CH₃ (OME₂) zu Methylal (OME₁) und Formaldehyd reagiert und Formaldehyd und Methanol zu Methylal (OME₁) reagieren; und ein destillatives Auftrennen in eine erste Fraktion, die Methylal (OME₁) enthält und die Reaktivdestillationseinheit RD2 als Kopfstrom KS_{RD2} verlässt, und eine zweite Fraktion, die Wasser enthält und die Reaktivdestillationseinheit RD2 als Sumpfstrom SS_{RD2} verlässt.

Das Mischen des aus der Destillationseinheit D1 abgezogenen Kopfstroms KS_{D1} mit dem methanolhaltigen Strom S_{MeOH} findet bevorzugt außerhalb der Reaktivdestillationseinheit RD2 statt und das dabei erhaltene Gemisch M1 wird anschließend in die Reaktivdestillationseinheit RD2 eingeleitet. Im Rahmen der vorliegenden Erfindung ist es allerdings auch möglich, dass der Kopfstrom KS_{D1} und der methanolhaltige Strom S_{MeOH} erst in der Reaktivdestillationseinheit RD2 miteinander gemischt werden.

Die in der ersten unabhängigen Ausführungsform des erfindungsgemäßen Verfahrens verwendete Reaktivdestillationseinheit RD2 (z.B. eine Reaktivdestillationskolonne) weist eine oder mehrere Reaktionszonen RZ und eine oder mehrere destillative Trennzonen auf. Die Reaktionszone RZ enthält einen sauren Katalysatoren (z.B. einen oder mehrere saure Feststoff-Katalysatoren, beispielsweise ein saure Gruppen aufweisendes lonenaustauscherharz (d.h. ein Kationenaustauscherharz), ein Zeolith, ein Aluminosilicat, ein Aluminiumoxid, ein Übergangsmetalloxid (das optional auf einem Trägermaterial vorliegt) oder ein Graphenoxid) für die Umsetzung von OME₂ zu Methylal (OME₁) und Formaldehyd (siehe obige Reaktionsgleichung 7) sowie die Umsetzung von Formaldehyd und Methanol zu Methylal (OME₁). Die destillative Trennzone enthält beispielsweise Einbauten für die destillative Trennung, insbesondere Böden, Füllkörper oder strukturierte Packungen, wie sie dem Fachmann allgemein bekannt sind. Die Immobilisierung des Katalysators in den Reaktionszonen RZ der Reaktivdestillationseinheit RD2 kann auf eine dem Fachmann bekannte Weise erfolgen, z.B. als regelloser Schüttfüllkörper; in Form von Katalysator-befüllten Drahtgewebekugeln oder als Katalysatorformkörper, die auf einem Boden in der Reaktionszone RZ angebracht sind. Sofern die Reaktivdestillationseinheit RD2 zwei oder mehr Reaktionszonen RZ enthält, kann es bevorzugt sein, dass zwischen zwei Reaktionszonen RZ jeweils eine destillative Trennzone vorliegt.

In der katalysatorhaltigen Reaktionszone RZ der Reaktivdestillationseinheit RD2 erfolgt eine chemische Umsetzung des Gemisches M1, wobei H₃C-O-(CH₂O)₂-CH₃ (OME₂) zu Methylal (OME₁) und Formaldehyd reagiert und Formaldehyd und Methanol zu Methylal (OME₁) reagieren. Außerdem erfolgt in der Reaktivdestillationseinheit RD2 ein destillatives Auftrennen in eine erste Fraktion, die Methylal (OME₁) und optional Methanol enthält und die Reaktivdestillationseinheit RD2 als Kopfstrom KS_{RD2} verlässt, und eine zweite Fraktion, die Wasser und optional überschüssiges Methanol, nicht umgesetztes Formaldehyd, Halbacetale der Formel HO-(CH₂O)ₙ-CH₃ n≥1 und/oder Glykole der Formel HO-(CH₂O)ₙ-H mit n≥1 enthält und die Reaktivdestillationseinheit RD2 als Sumpfstrom SS_{RD2} verlässt.

Die Reaktivdestillationseinheit RD2 ermöglicht
- die Gewinnung eines Kopfstroms KS_{RD2}, der im Wesentlichen Methylal und optional eine geringe Menge an Methanol enthält und daher als Methylalquelle für das Mischen mit der wasserhaltigen Formaldehydquelle S_{FA} zurückgeführt werden kann und
- die effektive Abtrennung des Wassers über den Sumpfstrom SS_{RD2}, der Wasser und optional überschüssiges Methanol, nicht umgesetztes Formaldehyd, Halbacetale der Formel HO-(CH₂O)ₙ-CH₃ n≥1 und/oder Glykole der Formel HO-(CH₂O)ₙ-H mit n≥1 enthält.

Wie oben erwähnt, reagieren in der katalysatorhaltigen Reaktionszone RZ der Reaktivdestillationseinheit RD2 Formaldehyd und Methanol zu Methylal (OME₁). Über das molare Verhältnis von Formaldehyd zu Methanol im Gemisch M1 kann gesteuert werden, ob (i) der Formaldehyd größtenteils oder sogar vollständig in OME₁ überführt wird und ein nicht umgesetzter Rest an Methanol verbleibt oder (ii) ein nicht umgesetzter Rest an Formaldehyd verbleibt. Bei Variante (i) enthält der Sumpfstrom SS_{RD2} neben dem Wasser bevorzugt noch Methanol und optional eine relativ geringe Menge an Formaldehyd, während bei Variante (ii) der Sumpfstrom SS_{RD2} neben dem Wasser bevorzugt noch Formaldehyd und optional eine relativ geringe Menge an Methanol enthält.

Bei Variante (i) enthält der wasserhaltige Sumpfstrom SS_{RD2} Methanol beispielsweise in einem Anteil von ≥ 0 Gew% bis 80 Gew%, bevorzugter ≥ 0 Gew% bis 30 Gew%. Bevorzugt beträgt in Summe der Anteil von Wasser und Methanol im Sumpfstrom SS_{RD2} mehr als 95 Gew%. Zudem können optional noch Anteile von Formaldehyd, H₃C-O-(CH₂O)₂-CH₃ (OME₂) und/oder Methylal (OME₁) in dem Sumpfstrom SS_{RD2} vorliegen, welche in Summe bevorzugt einen Anteil <5 Gew%, besonders bevorzugt einen Anteil <1 Gew% ausmachen.

Bei Variante (ii) enthält der wasserhaltige Sumpfstrom SS_{RD2} Formaldehyd beispielsweise in einem Anteil von ≥ 0 Gew% bis 60 Gew%, bevorzugter 25 Gew% bis 55 Gew %. Bevorzugt beträgt in Summe der Anteil von Wasser und Formaldehyd im Sumpfstrom SS_{RD2} mehr als 80 Gew%, bevorzugter mehr als 95 Gew%. Zudem können optional noch Anteile von unreagiertem MeOH, H₃C-O-(CH₂O)₂-CH₃ (OME₂) und/oder Methylal (OME₁) in dem Sumpfstrom SS_{RD2} vorliegen, welche in Summe bevorzugt einen Anteil <20 Gew% (wobei der Anteil von OME₂ im Sumpfstrom SS_{RD2} bevorzugt weniger als 5 Gew% beträgt), besonders bevorzugt einen Anteil <5 Gew% (wobei der Anteil von OME₂ im Sumpfstrom SS_{RD2} bevorzugt weniger als 2 Gew% beträgt) ausmachen. Beispielsweise enthält der wasserhaltige Sumpfstrom SS_{RD2} 25-55 Gew% Formaldehyd, wobei in Summe der Anteil von Wasser und Formaldehyd im Sumpfstrom mehr als 95 Gew% beträgt und der Anteil von OME₂ im Sumpfstrom SS_{RD2} weniger als 2 Gew% beträgt.

Geeignete Katalysatoren für die Umsetzung von H₃C-O-(CH₂O)₂-CH₃ (OME₂) zu Methylal (OME₁) und Formaldehyd und für die Umsetzung von Formaldehyd und Methanol zu Methylal (OME₁) sind dem Fachmann bekannt. Der Katalysator ist ein saurer Katalysator (z.B. ein oder mehrere saure Feststoff-Katalysatoren, beispielsweise ein saure Gruppen aufweisendes lonenaustauscherharz (d.h. ein Kationenaustauscherharz), ein Zeolith, ein Aluminosilicat, ein Aluminiumoxid, ein Übergangsmetalloxid (das optional auf einem Trägermaterial vorliegt) oder ein Graphenoxid).

Die Reaktivdestillationseinheit RD2 wird beispielsweise bei einem Druck im Bereich von 1-5 bar und einer Temperatur im Bereich von 40-140°C betrieben.

Beispielsweise wird das Gemisch M1 in einem Bereich, der oberhalb der katalysatorhaltigen Reaktionszone RZ liegt, in die Reaktivdestillationseinheit RD2 eingeleitet. Optional kann sich oberhalb dieses Einleitungsbereichs des Gemisches M1 eine weitere katalysatorhaltige Reaktionszone RZ befinden und in diese weitere Reaktionszone RZ wird ein Strom eingeleitet, der überwiegend (z.B. mindestens 80 Gew%) Formaldehyd, OME_{≥2} oder Trioxan oder ein Gemisch aus mindestens zwei dieser Komponenten enthält. Dies kann vorteilhaft sein, um einen Kopfstrom KS_{RD2} mit einem möglichst geringen Anteil an Methanol zu erhalten.

Optional kann aus der Reaktivdestillationseinheit RD2 neben dem Kopfstrom KS_{RD2} und dem Sumpfstrom SS_{RD2} noch mindestens ein Seitenstrom, beispielsweise ein MeOH-reicher Seitenstrom mit einem MeOH-Gehalt von mindestens 70 Gew%, abgezogen werden. Dieser Seitenstrom wird beispielsweise aus einem Bereich der Reaktivdestillationseinheit RD2 abgezogen, der zwischen der Reaktionszone RZ und dem Abzugsbereich des Sumpfstroms SS_{RD2} (d.h. dem Boden der Reaktivdestillationseinheit RD2) liegt.

In einer bevorzugten Ausführungsform wird zumindest ein Teil des aus der Reaktivdestillationseinheit RD2 abgezogenen Kopfstroms KS_{RD2} zurückgeführt und fungiert als methylalhaltiger Strom S_{OME1}, der mit dem formaldehyd- und wasserhaltigen Strom S_{FA} unter Erhalt des Reaktantengemisches M_{Reaktant} gemischt wird. Wie oben bereits erwähnt, erfolgt das Mischen der beiden Ströme bevorzugt vor dem Reaktor R1 und das dabei erhaltene Reaktantengemisch M_{Reaktant} wird anschließend in den Reaktor R1 eingeleitet. Alternativ ist es aber auch möglich, die beiden Ströme erst im Reaktor R1 miteinander zu mischen.

Sofern in dem aus der Reaktivdestillationseinheit RD2 abgezogenen Kopfstrom KS_{RD2} noch Methanol vorliegt, kann es beispielsweise vorteilhaft sein, wenn der Kopfstrom KS_{RD2} bei seiner Rückführung eine Destillationseinheit D4 passiert, in der das Methanol zumindest teilweise destillativ abgetrennt wird.

Bevorzugt passiert der aus der Reaktivdestillationseinheit RD2 abgezogene Kopfstrom KS_{RD2} bei seiner Rückführung einen Massenstromteiler, in dem ein Teil des Kopfstrom KS_{RD2} abgezweigt wird. Durch die Verwendung des Massenstromteilers lässt sich das Verhältnis an Methylal zu Formaldehyd im Reaktantengemisch M_{Reaktant} regulieren. Dies wiederum unterstützt die Einstellung eines konstanten Verhältnisses von Formaldehyd zu Methylal im Reaktantengemisch M_{Reaktant} und die Regulierung der Anteile an den längerkettigen Polyoxymethylendimethylethern OME_{≥3} im finalen Produkt. Das im Massenstromteiler abgezweigte Methylal stellt seinerseits ein potentiell interessantes Ausgangsmaterial für andere Prozesse dar und kann bis zur weiteren Verwendung gelagert werden. Massenstromteiler, mit denen sich Produktströme in zwei oder mehr Teilströme aufsplitten lassen, sind dem Fachmann bekannt.

Der in der ersten unabhängigen Ausführungsform des erfindungsgemäßen Verfahrens aus der Destillationseinheit D1 abgezogene Sumpfstrom SS_{D1} wird in eine Destillationseinheit D3 eingeleitet. Wie oben erwähnt, enthält der Sumpfstrom SS_{D1} Polyoxymethylendimethylether der Formeln H₃C-O-(CH₂O)₃₋₅-CH₃ (OME₃₋₅) und H₃C-O-(CH₂O)ₙ-CH₃ mit n≥6 (OME_{≥6}). In der Destillationseinheit D3 erfolgt ein destillatives Auftrennen dieser Polyoxymethylendimethylether in eine Fraktion, die die Destillationseinheit D3 als Kopfstrom KS_{D3} verlässt, und eine Fraktion, die die Destillationseinheit als Sumpfstrom SS_{D3} verlässt. Die für die destillative Trennung gewählten Bedingungen in der Destillationseinheit D3 können dem gewünschten Produktspektrum angepasst werden. Beispielsweise wird die Destillationseinheit D3 so betrieben, dass der aus der Destillationseinheit D3 abgezogene Kopfstrom KS_{D3} H₃C-O-(CH₂O)₃₋₅-CH₃ enthält und der Sumpfstrom SS_{D3} Polyoxymethylendimethylether der Formel H₃C-O-(CH₂O)ₙ-CH₃ mit n≥6 enthält. Die Destillationseinheit D3 enthält beispielsweise Einbauten für die destillative Trennung, insbesondere Böden, Füllkörper oder strukturierte Packungen, wie sie dem Fachmann allgemein bekannt sind. Die Destillationseinheit D3 wird beispielsweise bei einem Druck im Bereich von 0,05-3 bar und einer Temperatur im Bereich von 60-220°C betrieben. Sofern die Destillationseinheit bei einem reduzierten Druck (0,05 bar bis < 1,0 bar) betrieben wird, kann es gegebenenfalls vorteilhaft sein, Einbauten, die zu einem geringen Druckgefälle führen, zu verwenden. Bevorzugt ist die Destillationseinheit D3 katalysatorfrei (insbesondere frei von sauren Katalysatoren). Bevorzugt ist die Destillationseinheit D3 keine Reaktivdestillationseinheit.

Optional kann zumindest ein Teil des aus der Destillationseinheit D3 abgezogenen Sumpfstroms SS_{D3} zurückgeführt und mit dem formaldehyd- und wasserhaltigen Strom S_{FA} gemischt werden.

Wie oben beschrieben wurde, kann der aus der Reaktivdestillationseinheit RD2 abgezogene Sumpfstrom SS_{RD2} neben Wasser optional noch Formaldehyd enthalten (siehe die oben beschriebene Variante (ii)). Da Formaldehyd einer der Reaktanten des erfindungsgemäßen Verfahrens ist, kann es für diesen Fall vorteilhaft sein, den formaldehydhaltigen Sumpfstrom SS_{RD2} zurückzuführen und in eine Konzentratoreinheit FC einzuleiten, wobei in der Konzentratoreinheit ein Teil des Wassers entfernt wird und ein Strom die Konzentratoreinheit verlässt, der als Strom S_{FA} fungiert und mit dem methylalhaltigen Strom S_{OME1} unter Erhalt des Reaktantengemisches M_{Reaktant} gemischt wird. Vor dem Einleiten in die Konzentratoreinheit FC wird der zurückgeführte formaldehydhaltige Sumpfstrom SS_{RD2} bevorzugt mit einem formaldehydhaltigen Ausgangsmaterial (z.B. einer wässrigen Formaldehydlösung mit einem Formaldehydgehalt von mindestens 30 Gew%, bevorzugter mindestens 50 Gew%) unter Erhalt eines formaldehydhaltigen Gemisches M2 gemischt. Das formaldehydhaltige Gemisch M2 wird in die Konzentratoreinheit FC eingeleitet und ein Teil des Wassers wird in der Konzentratoreinheit FC entfernt, um die Konzentration an Formaldehyd zu erhöhen. Aus der Konzentratoreinheit FC wird ein Strom abgezogen, der als Formaldehydquelle S_{FA} fungiert und mit dem methylalhaltigen Strom S_{OME1} unter Erhalt des Reaktantengemisches M_{Reaktant} gemischt wird.

Geeignete Elemente einer Konzentratoreinheit sind dem Fachmann bekannt. Beispielsweise enthält die Konzentratoreinheit einen oder mehrere Filmverdampfer. Der Filmverdampfer ist beispielsweise ein Dünnschichtverdampfer, ein Wendelrohrverdampfer oder ein Fallfilmverdampfer. Hinsichtlich geeigneter Konzentratoreinheiten für die Erhöhung der Formaldehydkonzentration in wässrigen Formaldehydlösungen kann auch auf WO 03/040075 A2 und EP 1 688 168 A1 verwiesen werden.

In dieser Konstellation ergeben sich für die selektive Umwandlung von Formaldehyd und Methanol zu längerkettigen OMEs drei Hautprozessschritte. Die Aufkonzentration der Formaldehydquelle, die Reaktion von Formaldehyd mit dem rückgeführten Methylal zu längerkettigen OMEs und die reaktive Auftrennung des Produktgemisches in einen Produktstrom, der die längerkettigen OMEs enthält, einen Produktstrom der überwiegend Wasser enthält und optional einen Produktstrom der überwiegend Methylal enthält.

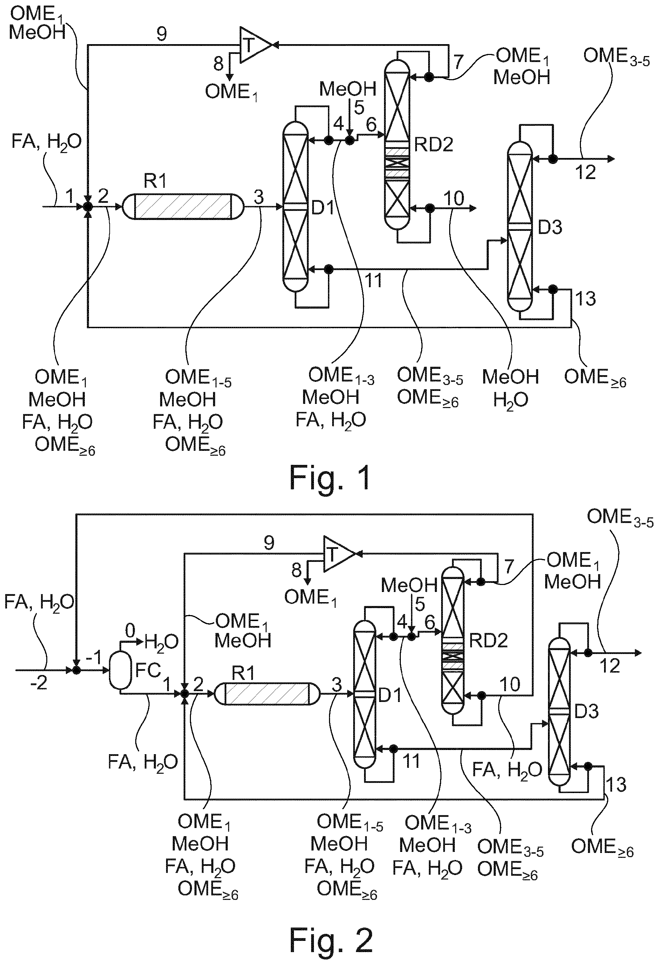

Eine beispielhafte Ausgestaltung der ersten unabhängigen Ausführungsform der vorliegenden Erfindung wird anhand der Figur 1 eingehender beschrieben.

Ein formaldehyd- und wasserhaltiger Strom S_{FA}, zugeführt über Leitung 1, wird mit einem methylalhaltigen Strom S_{OME1}, zugeführt über Leitung 9, gemischt. Wie nachfolgend noch eingehender beschrieben wird, handelt es sich bei dem methylalhaltigen Strom S_{OME1} um den Kopfstrom KS_{RD2}, der über Leitung 7 aus der Reaktivdestillationseinheit RD2 abgezogen wurde und bei seiner Rückführung einen Massenstromteiler T passiert. Optional kann der Strom S_{OME1} eine geringe Menge an Methanol (< 10 Gew%) enthalten. Optional können längerkettige OMEs (z.B. OME_{≥6}), die als Sumpfstrom SS_{D3} über Leitung 13 aus der Destillationseinheit D3 abgezogen werden, den Strömen S_{FA} und S_{OME1} beigemischt werden.

Durch das Mischen der Ströme S_{FA} und S_{OME1} (sowie optional SS_{D3}) wird ein Reaktantengemisch M_{Reaktant} erhalten, das über Leitung 2 in einen Reaktor R1, beispielsweise einen Festbettreaktor, eingeleitet wird. In dem Reaktor R1 reagieren Formaldehyd und OME₁ zu OME₂, OME₃₋₅ und OME_{≥6}. Es wird ein Produktgemisch M_{R1} erhalten, das OME₂, OME₃₋₅ und OME_{≥6} sowie OME₁, Formaldehyd, Methanol und Wasser enthält.

Über Leitung 3 wird das Produktgemisch M_{R}, aus dem Reaktor R1 abgezogen und in die Destillationseinheit D1 eingeleitet. In der Destillationseinheit D1 erfolgt ein destillatives Auftrennen des Produktgemisches M_{R1} in eine erste Fraktion, die Methylal (OME₁), H₃C-O-(CH₂O)₂-CH₃ (OME₂), Formaldehyd, Methanol und Wasser (und optional OME₃) enthält und die Destillationseinheit D1 über Leitung 4 als Kopfstrom KS_{D1} verlässt, sowie eine zweite Fraktion, die die Polyoxymethylendimethylether der Formeln H₃C-O-(CH₂O)₃₋₅-CH₃ (OME₃₋₅) und H₃C-O-(CH₂O)ₙ-CH₃ mit n≥6 (OME₂₆) enthält und die Destillationseinheit D1 über Leitung 11 als Sumpfstrom SS_{D1} verlässt.

Der aus der Destillationseinheit D1 über die Leitung 4 abgezogene Kopfstrom KS_{D1} wird mit einem methanolhaltigen Strom S_{MeOH}, zugeführt über Leitung 5, gemischt. Das dabei erhaltene Gemisch M1 wird über Leitung 6 in eine Reaktivdestillationseinheit RD2 eingeleitet. Das molare Verhältnis von Methanol zu Formaldehyd im Gemisch M1 wird so gewählt, dass der Formaldehyd in der Reaktivdestillationseinheit vollständig in OME₁ überführt wird und ein nicht umgesetzter Rest an Methanol verbleibt.

Die Reaktivdestillationseinheit RD2 weist katalysatorhaltige Reaktionszonen auf, in denen OME₂ (und optional OME₃, sofern der aus der Destillationseinheit D1 abgezogene Kopfstrom KS_{D}, noch einen gewissen Anteil an OME₃ enthielt) zu Methylal (OME₁) und Formaldehyd umgesetzt wird und Formaldehyd und Methanol zu Methylal (OME₁) umgesetzt werden. Außerdem erfolgt in der Reaktivdestillationseinheit RD2 ein destillatives Auftrennen in eine erste Fraktion, die Methylal (und optional Methanol) enthält und die Reaktivdestillationseinheit RD2 über Leitung 7 als Kopfstrom KS_{RD2} verlässt, und eine zweite Fraktion, die im Wesentlichen Wasser und Methanol enthält. Diese wasserhaltige Fraktion verlässt die Reaktivdestillationseinheit RD2 über Leitung 10 als Sumpfstrom SS_{RD2}.

Der über Leitung 7 aus der Reaktivdestillationseinheit RD2 abgezogene Kopfstrom KS_{RD2} wird zurückgeführt und fungiert als methylalhaltiger Strom S_{OME1}, der über Leitung 9 mit dem formaldehyd- und wasserhaltigen Strom S_{FA} (zugeführt über Leitung 1) unter Erhalt des Reaktantengemisches M_{Reaktant} gemischt wird. Bei seiner Rückführung passiert der Kopfstrom KS_{RD2} einen Massenstromteiler T, in dem ein Teil des Kopfstroms KS_{RD2} abgezweigt wird. Durch die Verwendung des Massenstromteilers lässt sich das Verhältnis von Formaldehyd zu Methylal im Reaktantengemisch M_{Reaktant} regulieren. Das im Massenstromteiler abgezweigte Methylal stellt seinerseits ein potentiell interessantes Ausgangsmaterial für andere Prozesse dar und kann bis zur weiteren Verwendung gelagert werden.

Der aus der Destillationseinheit D1 über Leitung 11 abgezogene Sumpfstrom SS_{D1} wird in eine Destillationseinheit D3 eingeleitet. Wie oben bereits erwähnt, enthält der Sumpfstrom SS_{D1} OME₃₋₅ und OME_{≥6}. In der Destillationseinheit D3 erfolgt ein destillatives Auftrennen dieser Polyoxymethylendimethylether in eine Fraktion, die OME₃₋₅ enthält und die Destillationseinheit D3 über Leitung 12 als Kopfstrom KS_{D3} verlässt, und eine Fraktion, die OME_{≥6} enthält und die Destillationseinheit D3 über Leitung 13 als Sumpfstrom SS_{D3} verlässt. Optional kann der Sumpfstrom SS_{D3} zurückgeführt und mit der wasserhaltigen Formaldehydquelle S_{FA} (Leitung 1) gemischt werden.

Eine weitere beispielhafte Ausgestaltung der ersten unabhängigen Ausführungsform des erfindungsgemäßen Verfahrens wird anhand der Figur 2 eingehender beschrieben. Die in Figur 2 veranschaulichte Prozessführung unterscheidet sich von der in Figur 1 veranschaulichten Prozessführung folgendermaßen:
- Im Gemisch M1 (erhalten durch das Mischen des Kopfstroms KS_{D1} (Leitung 4) mit dem methanolhaltigen Stroms S_{MeOH} (Leitung 5)) wird das molare Verhältnis von Methanol zu Formaldehyd so gewählt, dass das Methanol in der Reaktivdestillationseinheit RD2 weitestgehend in OME₁ überführt wird und ein nicht umgesetzter Rest an Formaldehyd als Bestandteil des wasserhaltigen Sumpfstroms SS_{RD2} über Leitung 10 aus der Reaktivdestillationseinheit RD2 abgezogen wird.
- Dieser Sumpfstrom SS_{RD2}, der neben Wasser noch Formaldehyd und optional eine geringe Menge an Methanol enthält, wird zurückgeführt und mit einer wässrigen Formaldehydlösung, zugeführt über Leitung -2, gemischt. Das dabei erhaltene Gemisch M2 wird über Leitung -1 in die Konzentratoreinheit FC, die beispielsweise einen oder mehrere Dünnschichtverdampfer enthält, eingeleitet und ein Teil des Wassers wird über Leitung 0 entfernt, um die Konzentration an Formaldehyd zu erhöhen. Aus der Konzentratoreinheit FC wird über Leitung 1 ein Strom abgezogen, der als Formaldehydquelle S_{FA} fungiert.

Hinsichtlich aller weiteren Merkmale der in Figur 2 veranschaulichten beispielhaften Ausgestaltung der ersten unabhängigen Ausführungsform der vorliegenden Erfindung kann auf die obige Beschreibung zu Figur 1 verwiesen werden.

In einem Beispiel des in Figur 2 veranschaulichten Verfahrens wurde der Reaktor R1 bei 100°C und 10 bar betrieben. Als saurer Katalysator wurde Amberlyst^{®} 46 verwendet.

Das dem Reaktor R1 zugeführte Reaktantengemisch M_{Reaktant} und das im Reaktor R1 erhaltene Produktgemisch M_{R1} wiesen die in der folgenden Tabelle 1 angegebenen Zusammensetzungen auf.

**Tabelle 1: Zusammensetzungen des Reaktantengemisches M_{Reaktant} und des im Reaktor R1 erhaltenen Produktgemisches M_{R1}**

| Zusammensetzung Reaktantengemisch M_{Reaktant} | Zusammensetzung Produktgemisch M_{R1} |
|---|---|
| 36 Gew% Formaldehyd | 24 Gew% OME₁ |
| 4 Gew% Wasser | 17 Gew% OME₂ |
| 60 Gew% OME₁ | 24 Gew% OME₃₋₅ |
| | 6 Gew% OME_{≥6} |
| | 19 Gew% Formaldehyd |
| | 2 Gew% Wasser |
| | 8 Gew% Methanol |

Nach der destillativen Auftrennung in einen Kopfstrom KS_{D1} und einen Sumpfstrom SS_{D1} in der Destillationseinheit D1 wurde der Kopfstrom KS_{D1} mit einem methanolhaltigen Strom S_{MeOH} unter Erhalt eines Gemisches M1 zusammengeführt und dieses Gemisch M1 in eine Reaktivdestillationseinheit RD2 eingeleitet. Als saurer Katalysator in der Reaktivdestillationseinheit RD2 wurde Amberlyst^{®} 46 verwendet. Das Gemisch M1 wurde oberhalb der katalysatorhaltigen Reaktionszone eingeleitet. Während der Destillation stellte sich eine Destillattemperatur von 41°C ein, was der Siedetemperatur des azeotropen Gemisches aus OME₁ und Methanol entspricht. Im Sumpf der Reaktivdestillationseinheit RD2 wurde eine Temperatur leicht oberhalb von 100°C erreicht. RD2 wurde bei Umgebungsdruck betrieben.

Die Zusammensetzungen des in die Reaktivdestillationseinheit RD2 eingeleiteten Gemischs M1 sowie des in RD2 erhaltenen Kopfstroms KS_{RD2} und Sumpfstroms SS_{RD2} werden in der nachfolgenden Tabelle 2 angegeben.

**Tabelle 2: Zusammensetzungen des in die Reaktivdestillationseinheit RD2 eingeleiteten Gemischs M1 sowie des in RD2 erhaltenen Kopfstroms KS_{RD2} und Sumpfstroms SS_{RD2}**

| Zusammensetzung Gemisch M1 | Zusammensetzung Kopfstrom KS_{RD2} | Zusammensetzung Sumpfstrom SS_{RD2} |
|---|---|---|
| 18 Gew% Formaldehyd | 94 Gew% OME1 | 44 Gew% Formaldehyd |
| 1 Gew% Wasser | 6 Gew% Methanol | 56 Gew% Wasser |
| 38 Gew% Methanol | | |
| 23 Gew% OME₁ | | |
| 16 Gew% OME₂ | | |
| 3 Gew% OME₃ | | |

Der Sumpfstrom SS_{RD2}, der im Wesentlichen aus Wasser und Formaldehyd besteht, kann zurückgeführt und somit Teil der wasserhaltigen Formaldehydausgangsquelle S_{FA} werden. Die Verwendung zusätzlicher Wasserabtrenneinheiten ist nicht erforderlich.

Das im Gemisch M1 vorhandene OME₂ konnte im Wesentlichen vollständig umgesetzt werden, so dass Sumpfstrom SS_{RD2} und Kopfstrom KS_{RD2} im Wesentlichen frei von OME₂ sind.

Nachfolgend wird die zweite unabhängige Ausführungsform der Erfindung eingehender beschrieben.

Wie oben bereits beschrieben, wird sowohl in der ersten wie auch in der zweiten unabhängigen Ausführungsform der vorliegenden Erfindung zunächst das Reaktantengemisch M_{Reaktant} in dem Reaktor R1 zu dem Produktgemisch M_{R1}, das Polyoxymethylendimethylether der Formeln H₃C-O-(CH₂O)₂-CH₃ (OME₂), H₃C-O-(CH₂O)₃₋₅-CH₃ (OME₃₋₅) und H₃C-O-(CH₂O)ₙ-CH₃ mit n≥6 (OME₂₆) sowie Formaldehyd, Methylal (OME₁), Methanol und Wasser enthält, umgesetzt.

In der zweiten unabhängigen Ausführungsform der vorliegenden Erfindung wird das im Reaktor R1 erhaltene Produktgemisch M_{R1} mit einem methanolhaltigen Strom S_{MeOH} gemischt. Das dabei erhaltene Gemisch M1 wird in mindestens einer Reaktionszone RZ einer Reaktivdestillationseinheit RD1 in Gegenwart eines Katalysators umgesetzt, wobei H₃C-O-(CH₂O)₂-CH₃ (OME₂) zu Methylal (OME₁) und Formaldehyd reagiert und Formaldehyd und Methanol zu Methylal (OME₁) reagieren; und es erfolgt ein destillatives Auftrennen in eine erste Fraktion, die Wasser, Methanol, Formaldehyd, Methylal (OME₁) und H₃C-O-(CH₂O)₂-CH₃ (OME₂) enthält und die Reaktivdestillationseinheit RD1 als Kopfstrom KS_{RD1} verlässt, und eine zweite Fraktion, die die Polyoxymethylendimethylether der Formeln H₃C-O-(CH₂O)₃₋₅-CH₃ (OME₃₋₅) und H₃C-O-(CH₂O)ₙ-CH₃ mit n≥6 (OME₂₆) enthält und die Reaktivdestillationseinheit RD1 als Sumpfstrom SS_{RD1} verlässt.

Das Mischen des aus dem Reaktor R1 abgezogenen Produktgemisches M_{R1} mit dem methanolhaltigen Strom S_{MeOH} findet bevorzugt außerhalb der Reaktivdestillationseinheit RD1 statt und das dabei erhaltene Gemisch M1 wird anschließend in die Reaktivdestillationseinheit RD1 eingeleitet. Im Rahmen der vorliegenden Erfindung ist es allerdings auch möglich, dass das Produktgemisch M_{R1} und der methanolhaltige Strom S_{MeOH} erst in der Reaktivdestillationseinheit RD1 miteinander gemischt werden.

Optional kann der methanolhaltige Strom S_{MeOH} noch weitere Komponenten (z.B. Formaldehyd, Wasser, OME₁, OME_{≥2} oder Trioxan) enthalten. Bevorzugt ist jedoch, dass der methanolhaltige Strom S_{MeOH} mindestens 80 Gew% Methanol, bevorzugter mindestens 90 Gew% Methanol enthält.

Die in der zweiten unabhängigen Ausführungsform des erfindungsgemäßen Verfahrens verwendete Reaktivdestillationseinheit RD1 (z.B. eine Reaktivdestillationskolonne) weist eine oder mehrere Reaktionszonen RZ und eine oder mehrere destillative Trennzonen auf. Die Reaktionszone RZ enthält einen sauren Katalysatoren (z.B. einen oder mehrere saure Feststoff-Katalysatoren, beispielsweise ein saure Gruppen aufweisendes lonenaustauscherharz (d.h. ein Kationenaustauscherharz), ein Zeolith, ein Aluminosilicat, ein Aluminiumoxid, ein Übergangsmetalloxid (das optional auf einem Trägermaterial vorliegt) oder ein Graphenoxid) für die Umsetzung von OME₂ zu Methylal (OME₁) und Formaldehyd (siehe obige Reaktionsgleichung 7) sowie die Umsetzung von Formaldehyd und Methanol zu Methylal (OME₁). Die destillative Trennzone enthält beispielsweise Einbauten für die destillative Trennung, insbesondere Böden, Füllkörper oder strukturierte Packungen, wie sie dem Fachmann allgemein bekannt sind. Die Immobilisierung des Katalysators in den Reaktionszonen RZ der Reaktivdestillationseinheit RD1 kann auf eine dem Fachmann bekannte Weise erfolgen, z.B. als regelloser Schüttfüllkörper; in Form von Katalysator-befüllten Drahtgewebekugeln oder als Katalysatorformkörper, die auf einem Boden in der Reaktionszone RZ angebracht sind. Sofern die Reaktivdestillationseinheit RD1 zwei oder mehr Reaktionszonen RZ enthält, kann es bevorzugt sein, dass zwischen zwei Reaktionszonen RZ jeweils eine destillative Trennzone vorliegt.

In der katalysatorhaltigen Reaktionszone RZ der Reaktivdestillationseinheit RD1 erfolgt eine chemische Umsetzung des Gemisches M1, wobei H₃C-O-(CH₂O)₂-CH₃ (OME₂) zu Methylal (OME₁) und Formaldehyd reagiert und Formaldehyd und Methanol zu Methylal (OME₁) reagieren. Außerdem erfolgt in der Reaktivdestillationseinheit RD1 ein destillatives Auftrennen in eine erste Fraktion, die Wasser, Methanol, Formaldehyd, Methylal (OME₁) und H₃C-O-(CH₂O)₂-CH₃ (OME₂) und optional Halbacetale der Formel HO-(CH₂O)ₙ-CH₃ n≥1 und/oder Glykole der Formel HO-(CH₂O)ₙ-H mit n≥1 enthält und die Reaktivdestillationseinheit RD1 als Kopfstrom KS_{RD1} verlässt, und eine zweite Fraktion, die die Polyoxymethylendimethylether der Formeln H₃C-O-(CH₂O)₃₋₅-CH₃ (OME₃₋₅) und H₃C-O-(CH₂O)ₙ-CH₃ mit n≥6 (OME₂₆) enthält und die Reaktivdestillationseinheit RD1 als Sumpfstrom SS_{RD1} verlässt.

Durch die Reaktivdestillationseinheit RD1 und die der Reaktivdestillationseinheit RD1 nachgeschaltete Destillationseinheit D2 (die nachfolgend noch eingehender beschrieben wird) lässt sich Folgendes realisieren:
- Aus der Destillationseinheit D2 lässt sich ein Kopfstrom KS_{D2} abziehen, der im Wesentlichen Methylal und optional eine geringe Menge an Methanol enthält und daher als Methylalquelle für das Mischen mit der wasserhaltigen Formaldehydquelle S_{FA} zurückgeführt werden kann.
- Das Wasser lässt sich effizient abtrennen über den Sumpfstrom SS_{D2} der Destillationseinheit D2.

Geeignete Katalysatoren für die Umsetzung von H₃C-O-(CH₂O)₂-CH₃ (OME₂) zu Methylal (OME₁) und Formaldehyd und für die Umsetzung von Formaldehyd und Methanol zu Methylal (OME₁) in den Reaktionszonen RZ der Reaktivdestillationseinheit RD1 sind dem Fachmann bekannt. Der Katalysator ist ein saurer Katalysator (z.B. ein oder mehrere saure Feststoff-Katalysatoren, beispielsweise ein saure Gruppen aufweisendes lonenaustauscherharz (d.h. ein Kationenaustauscherharz), ein Zeolith, ein Aluminosilicat, ein Aluminiumoxid, ein Übergangsmetalloxid (das optional auf einem Trägermaterial vorliegt) oder ein Graphenoxid).

Die Reaktivdestillationseinheit RD1 wird beispielsweise bei einem Druck im Bereich von 1-15 bar und einer Temperatur im Bereich von 60-250°C betrieben.

Bevorzugt wird das Gemisch M1 in einem Bereich, der unterhalb der katalysatorhaltigen Reaktionszone RZ liegt, in die Reaktivdestillationseinheit RD1 eingeleitet. Sofern die Reaktivdestillationseinheit RD1 mehrere Reaktionszonen RZ aufweist, ist bevorzugt, dass das Gemisch M1 in einem Bereich in die Reaktivdestillationseinheit RD1 eingeleitet wird, der unterhalb aller in RD1 vorliegenden Reaktionszonen RZ liegt.

Wie oben erwähnt, reagieren in der katalysatorhaltigen Reaktionszone RZ der Reaktivdestillationseinheit RD1 Formaldehyd und Methanol zu Methylal (OME₁). Über das molare Verhältnis von Formaldehyd zu Methanol im Gemisch M1 kann gesteuert werden, ob (i) der Formaldehyd größtenteils oder sogar vollständig in OME₁ überführt wird und ein nicht umgesetzter Rest an Methanol verbleibt oder (ii) ein nicht umgesetzter Rest an Formaldehyd verbleibt.

Wie oben bereits erwähnt, enthält der aus der Reaktivdestillationseinheit RD1 abgezogene Kopfstrom KS_{RD1} Wasser, Methanol, Formaldehyd, Methylal (OME₁) und H₃C-O-(CH₂O)₂-CH₃ (OME₂). Sowohl bei Variante (i) als auch bei Variante (ii) weist der Kopfstrom KS_{RD1} üblicherweise einen sehr geringen Anteil an OME₂ auf, z.B. weniger als 5 Gew%, bevorzugter weniger als 2 Gew%. Bei Variante (i) weist der Kopfstrom KS_{RD1} einen sehr geringen Anteil an Formaldehyd auf, z.B. weniger als 5 Gew%. Bei Variante (ii) weist der Kopfstrom KSRD1 einen geringen Anteil an Methanol auf, z.B. weniger als 5 Gew%.

Der aus der Reaktivdestillationseinheit abgezogene Kopfstrom KS_{RD1} wird in eine Destillationseinheit D2 eingeleitet und es erfolgt ein destillatives Auftrennen in eine erste Fraktion, die Methylal enthält und die Destillationseinheit D2 als Kopfstrom KS_{D2} verlässt, und eine zweite Fraktion, die Wasser enthält und die Destillationseinheit D2 als Sumpfstrom SS_{D2} verlässt.

Die Destillationseinheit D2 ist beispielsweise katalysatorfrei (insbesondere frei von sauren Katalysatoren). Alternativ ist es im Rahmen der vorliegenden Erfindung auch möglich, dass die Destillationseinheit D2 eine Reaktivdestillationseinheit ist, die eine oder mehrere Reaktionszonen RZ und eine oder mehrere destillative Trennzonen aufweist. Die Reaktionszone RZ enthält einen oder mehrere saure Katalysatoren (z.B. einen oder mehrere saure Feststoff-Katalysatoren, beispielsweise ein saure Gruppen aufweisendes lonenaustauscherharz (d.h. ein Kationenaustauscherharz), ein Zeolith, ein Aluminosilicat, ein Aluminiumoxid, ein Übergangsmetalloxid (das optional auf einem Trägermaterial vorliegt) oder ein Graphenoxid).

Die Destillationseinheit D2 wird beispielsweise bei einem Druck im Bereich von 1-5 bar und einer Temperatur im Bereich von 40-140°C betrieben.

In der Destillationseinheit D2 erfolgt ein destillatives Auftrennen in eine erste Fraktion, die Methylal enthält und die Destillationseinheit D2 als Kopfstrom KS_{D2} verlässt, und eine zweite Fraktion, die Wasser enthält und die Destillationseinheit D2 als Sumpfstrom SS_{D2} verlässt.

Der Anteil an OME₂ im Sumpfstrom SS_{D2} ist üblicherweise sehr gering, z.B. weniger als 5 Gew%, bevorzugt weniger als 2 Gew%, noch bevorzugter weniger als 1 Gew%.

Kam die oben beschriebene Variante (i) zum Einsatz (d.h. Überschuss an Methanol, so dass der aus der Reaktivdestillationseinheit RD1 abgezogene Kopfstrom KS_{RD1} einen sehr geringen Anteil an Formaldehyd aufwies), so enthält der Sumpfstrom SS_{D2} beispielsweise Methanol in einem Anteil von ≥ 0 Gew% bis 80 Gew%, bevorzugter ≥ 0 Gew% bis 30 Gew%. Bevorzugt beträgt in Summe der Anteil von Wasser und Methanol im Sumpfstrom SS_{D2} mehr als 95 Gew%.

Optional enthält der Sumpfstrom SS_{D2} noch Formaldehyd, H₃C-O-(CH₂O)₂-CH₃ (OME₂) und/oder Methylal (OME₁), welche in Summe bevorzugt einen Anteil <5 Gew%, besonders bevorzugt einen Anteil <1 Gew% ausmachen.

Kam die oben beschriebene Variante (ii) zum Einsatz (d.h. größerer Anteil an nicht umgesetztem Formaldehyd in dem aus der Reaktivdestillationseinheit RD1 abgezogenen Kopfstrom KS_{RD1}), so enthält der Sumpfstrom SS_{D2} beispielsweise Formaldehyd in einem Anteil von ≥ 0 Gew% bis 60 Gew%, bevorzugter 25 Gew% bis 55 Gew%. Bevorzugt beträgt in Summe der Anteil von Wasser und Formaldehyd im Sumpfstrom SS_{D2} mehr als 80 Gew%, bevorzugter mehr als 95 Gew%. Optional enthält der Sumpfstrom SS_{RD2} noch MeOH, H₃C-O-(CH₂O)₂-CH₃ (OME₂) und/oder Methylal (OME₁), welche in Summe bevorzugt einen Anteil <20 Gew% (wobei der Anteil von OME₂ im Sumpfstrom SS_{D2} bevorzugt weniger als 5 Gew% beträgt), besonders bevorzugt einen Anteil <5 Gew% (wobei der Anteil von OME₂ im Sumpfstrom SS_{D2} bevorzugt weniger als 2 Gew% beträgt) ausmachen. Beispielsweise enthält der wasserhaltige Sumpfstrom SS_{D2} 25-55 Gew% Formaldehyd, wobei in Summe der Anteil von Wasser und Formaldehyd im Sumpfstrom mehr als 95 Gew% beträgt und der Anteil von OME₂ im Sumpfstrom SS_{D2} weniger als 2 Gew% beträgt.

In einer bevorzugten Ausführungsform wird zumindest ein Teil des aus der Destillationseinheit D2 abgezogenen Kopfstroms KS_{D2} zurückgeführt und fungiert als methylalhaltiger Strom S_{OME1}, der mit dem formaldehyd- und wasserhaltigen Strom S_{FA} unter Erhalt des Reaktantengemisches M_{Reaktant} gemischt wird. Wie oben bereits erwähnt, erfolgt das Mischen der beiden Ströme bevorzugt vor dem Reaktor R1 und das dabei erhaltene Reaktantengemisch M_{Reaktant} wird anschließend in den Reaktor R1 eingeleitet. Alternativ ist es aber auch möglich, die beiden Ströme erst im Reaktor R1 miteinander zu mischen.

Sofern in dem aus der Destillationseinheit D2 abgezogenen Kopfstrom KS_{D2} noch Methanol vorliegt, kann es beispielsweise vorteilhaft sein, wenn der Kopfstrom KS_{D2} bei seiner Rückführung eine Destillationseinheit D4 passiert, in der das Methanol zumindest teilweise destillativ abgetrennt wird.

Bevorzugt passiert der aus der Destillationseinheit D2 abgezogene Kopfstrom KS_{D2} bei seiner Rückführung einen Massenstromteiler, in dem ein Teil des Kopfstroms KS_{D2} abgezweigt wird. Durch die Verwendung des Massenstromteilers lässt sich das Verhältnis an Methylal zu Formaldehyd im Reaktantengemisch M_{Reaktant} regulieren. Dies wiederum unterstützt die Einstellung eines konstanten Verhältnisses von Formaldehyd zu Methylal im Reaktantengemisch M_{Reaktant} und die Regulierung der Anteile an den längerkettigen Polyoxymethylendimethylethern OME_{≥3} im finalen Produkt. Das im Massenstromteiler abgezweigte Methylal stellt seinerseits ein potentiell interessantes Ausgangsmaterial für andere Prozesse dar und kann bis zur weiteren Verwendung gelagert werden. Massenstromteiler, mit denen sich Produktströme in zwei oder mehr Teilströme aufsplitten lassen, sind dem Fachmann bekannt.

Der in der zweiten unabhängigen Ausführungsform des erfindungsgemäßen Verfahrens aus der Reaktivdestillationseinheit RD1 abgezogene Sumpfstrom SS_{RD1} wird in eine Destillationseinheit D3 eingeleitet. Wie oben erwähnt, enthält der Sumpfstrom SS_{RD1} Polyoxymethylendimethylether der Formeln H₃C-O-(CH₂O)₃₋₅-CH₃ (OME₃₋₅) und H₃C-O-(CH₂O)ₙ-CH₃ mit n≥6 (OME_{≥6}). In der Destillationseinheit D3 erfolgt ein destillatives Auftrennen dieser Polyoxymethylendimethylether in eine Fraktion, die die Destillationseinheit D3 als Kopfstrom KS_{D3} verlässt, und eine Fraktion, die die Destillationseinheit als Sumpfstrom SS_{D3} verlässt. Die für die destillative Trennung gewählten Bedingungen in der Destillationseinheit D3 können dem gewünschten Produktspektrum angepasst werden. Beispielsweise wird die Destillationseinheit D3 so betrieben, dass der aus der Destillationseinheit D3 abgezogene Kopfstrom KS_{D3} H₃C-O-(CH₂O)₃₋₅-CH₃ enthält und der Sumpfstrom SS_{D3} Polyoxymethylendimethylether der Formel H₃C-O-(CH₂O)ₙ-CH₃ mit n≥6 enthält. Die Destillationseinheit D3 enthält beispielsweise Einbauten für die destillative Trennung, insbesondere Böden, Füllkörper oder strukturierte Packungen, wie sie dem Fachmann allgemein bekannt sind. Die Destillationseinheit D3 wird beispielsweise bei einem Druck im Bereich von 0,05-3 bar und einer Temperatur im Bereich von 60-220°C betrieben. Sofern die Destillationseinheit bei einem reduzierten Druck (0,05 bar bis < 1,0 bar) betrieben wird, kann es gegebenenfalls vorteilhaft sein, Einbauten, die zu einem geringen Druckgefälle führen, zu verwenden. Bevorzugt ist die Destillationseinheit D3 katalysatorfrei (insbesondere frei von sauren Katalysatoren). Bevorzugt ist die Destillationseinheit D3 keine Reaktivdestillationseinheit.

Wie in der ersten unabhängigen Ausführungsform kann auch in der zweiten unabhängigen Ausführungsform der vorliegenden Erfindung optional zumindest ein Teil des aus der Destillationseinheit D3 abgezogenen Sumpfstroms SS_{D3} zurückgeführt und mit dem formaldehyd- und wasserhaltigen Strom S_{FA} gemischt werden.

Wie oben beschrieben wurde, kann der aus der Destillationseinheit D2 abgezogene Sumpfstrom SS_{D2} neben Wasser optional noch Formaldehyd enthalten (siehe die oben beschriebene Variante (ii)). Da Formaldehyd einer der Reaktanten des erfindungsgemäßen Verfahrens ist, kann es für diesen Fall vorteilhaft sein, den formaldehydhaltigen Sumpfstrom SS_{D2} zurückzuführen und in eine Konzentratoreinheit FC einzuleiten, wobei in der Konzentratoreinheit ein Teil des Wassers entfernt wird und ein Strom die Konzentratoreinheit verlässt, der als Strom S_{FA} fungiert und mit dem methylalhaltigen Strom S_{OME1} unter Erhalt des Reaktantengemisches M_{Reaktant} gemischt wird. Vor dem Einleiten in die Konzentratoreinheit FC wird der zurückgeführte formaldehydhaltige Sumpfstrom SS_{D2} bevorzugt mit einem formaldehydhaltigen Ausgangsmaterial (z.B. einer wässrigen Formaldehydlösung mit einem Formaldehydgehalt von mindestens 30 Gew%, bevorzugter mindestens 50 Gew%) unter Erhalt eines formaldehydhaltigen Gemisches M2 gemischt. Das formaldehydhaltige Gemisch M2 wird in die Konzentratoreinheit FC eingeleitet und ein Teil des Wassers wird in der Konzentratoreinheit FC entfernt, um die Konzentration an Formaldehyd zu erhöhen. Aus der Konzentratoreinheit FC wird ein Strom abgezogen, der als Formaldehydquelle S_{FA} fungiert und mit dem methylalhaltigen Strom S_{OME1} unter Erhalt des Reaktantengemisches M_{Reaktant} gemischt wird.

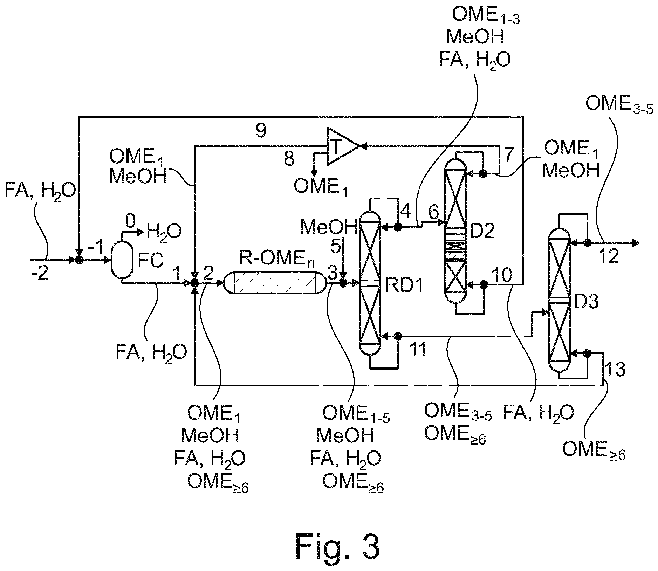

Eine beispielhafte Ausgestaltung der zweiten unabhängigen Ausführungsform der vorliegenden Erfindung wird anhand der Figur 3 eingehender beschrieben.

Eine wässrige Formaldehydlösung, zugeführt über Leitung -2, und ein aus der Destillationseinheit D2 zurückgeführter Sumpfstrom SS_{D2}, der neben Wasser noch Formaldehyd und optional Methanol enthält, werden gemischt. Das dabei erhaltene Gemisch M2 wird über Leitung -1 in die Konzentratoreinheit FC, die beispielsweise einen oder mehrere Dünnschichtverdampfer enthält, eingeleitet und ein Teil des Wassers wird über Leitung 0 entfernt, um die Konzentration an Formaldehyd zu erhöhen. Aus der Konzentratoreinheit FC wird über Leitung 1 ein Strom abgezogen, der als Formaldehydquelle S_{FA} fungiert.

Die Formaldehydquelle S_{FA}, zugeführt über Leitung 1, wird mit einem methylalhaltigen Strom S_{OME1}, zugeführt über Leitung 9, gemischt. Bei dem methylalhaltigen Strom S_{OME1} handelt es sich um den Kopfstrom KS_{D2}, der über Leitung 7 aus der Destillationseinheit D2 abgezogen wurde und bei seiner Rückführung einen Massenstromteiler T passiert. Optional kann der Strom S_{OME1} eine geringe Menge an Methanol (< 10 Gew%) enthalten. Optional können längerkettige OMEs (z.B. OME_{≥6}), die als Sumpfstrom SS_{D3} über Leitung 13 aus der Destillationseinheit D3 abgezogen werden, den Strömen S_{FA} und S_{OME1} beigemischt werden.

Durch das Mischen der Ströme S_{FA} und S_{OME1} (sowie optional SS_{D3}) wird ein Reaktantengemisch M_{Reaktant} erhalten, das über Leitung 2 in einen Reaktor R1, beispielsweise einen Festbettreaktor, eingeleitet wird. In dem Reaktor R1 reagieren Formaldehyd und OME₁ in Anwesenheit eines sauren Katalysators zu OME₂, OME₃₋₅ und OME_{≥6}. Es wird ein Produktgemisch M_{R1} erhalten, das OME₂, OME₃₋₅ und OME_{≥6} sowie OME₁, Formaldehyd, Methanol und Wasser enthält.

Über Leitung 3 wird das Produktgemisch M_{R1} aus dem Reaktor R1 abgezogen und mit einem methanolhaltigen Strom S_{MeOH}, zugeführt über Leitung 5, gemischt. Das dabei erhaltene Gemisch M1 wird in eine Reaktivdestillationseinheit RD1 eingeleitet. Die Reaktivdestillationseinheit RD1 weist mehrere Reaktionszonen, in denen jeweils ein saurer Katalysator vorliegt, und destillative Trennzonen auf. Das Gemisch M1 wird unterhalb der katalysatorhaltigen Reaktionszonen RZ in die Reaktivdestillationseinheit RD1 eingeleitet. Das molare Verhältnis von Methanol zu Formaldehyd im Gemisch M1 wird so gewählt, dass das Methanol in der Reaktivdestillationseinheit RD1 weitestgehend in OME₁ überführt wird und der aus RD1 abgezogene Kopfstrom SS_{RD1} daher einen relativ geringen Anteil an Methanol aufweist.

In den katalysatorhaltigen Reaktionszonen der Reaktivdestillationseinheit RD1 wird OME₂ zu Methylal (OME₁) und Formaldehyd umgesetzt wird und außerdem werden Formaldehyd und Methanol zu Methylal (OME₁) umgesetzt. Außerdem erfolgt in der Reaktivdestillationseinheit RD1 ein destillatives Auftrennen in eine erste Fraktion, die Wasser, Methanol, Formaldehyd, Methylal (OME₁) und H₃C-O-(CH₂O)₂-CH₃ (OME₂) enthält und die Reaktivdestillationseinheit RD1 als Kopfstrom KS_{RD1} verlässt, und eine zweite Fraktion, die die Polyoxymethylendimethylether der Formeln H₃C-O-(CH₂O)₃₋₅-CH₃ (OME₃₋₅) und H₃C-O-(CH₂O)ₙ-CH₃ mit n≥6 (OME₂₆) enthält und die Reaktivdestillationseinheit RD1 als Sumpfstrom SS_{RD1} verlässt.

Über Leitung 6 wird der aus RD1 abgezogene Kopfstrom KS_{RD1} in eine katalysatorfreie Destillationseinheit D2 eingeleitet. In dieser Destillationseinheit D2 erfolgt ein destillatives Auftrennen in eine erste Fraktion, die Methylal enthält und die Destillationseinheit D2 als Kopfstrom KS_{D2} verlässt, und eine zweite Fraktion, die Wasser und Formaldehyd enthält und die Destillationseinheit D2 als Sumpfstrom SS_{D2} verlässt.

Der über Leitung 7 aus der Destillationseinheit D2 abgezogene Kopfstrom KS_{D2} wird zurückgeführt und fungiert als methylalhaltiger Strom S_{OME1}, der über Leitung 9 mit der wässrigen Formaldehydlösung (zugeführt über Leitung -2) gemischt wird. Bei seiner Rückführung passiert der Kopfstrom KS_{D2} einen Massenstromteiler T, in dem ein Teil des Kopfstroms KS_{D2} abgezweigt wird. Durch die Verwendung des Massenstromteilers lässt sich das Verhältnis von Formaldehyd zu Methylal im Reaktantengemisch M_{Reaktant} regulieren. Das im Massenstromteiler abgezweigte Methylal stellt seinerseits ein potentiell interessantes Ausgangsmaterial für andere Prozesse dar und kann bis zur weiteren Verwendung gelagert werden.

Der über Leitung 10 aus der Destillationseinheit D2 abgezogene wässrige, formaldehydhaltige Sumpfstrom SS_{D2}, der im Wesentlichen aus Wasser und Formaldehyd besteht, wird zurückgeführt und mit der wässrigen Formaldehydausgangslösung, zugeführt über Leitung -2, gemischt.

Der aus der Reaktivdestillationseinheit RD1 über Leitung 11 abgezogene Sumpfstrom SS_{RD1} wird in eine Destillationseinheit D3 eingeleitet. Der Sumpfstrom SS_{D1} enthält OME₃₋₅ und OME_{≥6}. In der Destillationseinheit D3 erfolgt ein destillatives Auftrennen dieser Polyoxymethylendimethylether in eine Fraktion, die OME₃₋₅ enthält und die Destillationseinheit D3 über Leitung 12 als Kopfstrom KS_{D3} verlässt, und eine Fraktion, die OME_{≥6} enthält und die Destillationseinheit D3 über Leitung 13 als Sumpfstrom SS_{D3} verlässt. Optional kann der Sumpfstrom SS_{D3} zurückgeführt und mit der wasserhaltigen Formaldehydquelle S_{FA} (Leitung 1) gemischt werden.

## Patentansprüche

1. Verfahren zur Herstellung von Polyoxymethylendimethylethern, folgende Schritte umfassend:
- Mischen eines Stroms S_{FA}, der eine wasserhaltige Formaldehydquelle enthält, mit einem Strom S_{OME1}, der Methylal enthält, unter Erhalt eines Reaktantengemisches M_{Reaktant},
- Umsetzen des Reaktantengemisches M_{Reaktant} in einem Reaktor R1 unter Erhalt eines Produktgemisches M_{R1}, das Polyoxymethylendimethylether der Formeln H₃C-O-(CH₂O)₂-CH₃, H₃C-O-(CH₂O)₃₋₅-CH₃ und H₃C-O-(CH₂O)ₙ-CH₃ mit n≥6 sowie Formaldehyd, Methylal, Methanol und Wasser enthält,
- Einleiten des Produktgemisches M_{R1} in eine Destillationseinheit D1 und destillatives Auftrennen des Produktgemisches M_{R1} in eine erste Fraktion, die Methylal, H₃C-O-(CH₂O)₂-CH₃, Formaldehyd, Methanol und Wasser enthält und die Destillationseinheit D1 als Kopfstrom KS_{D1} verlässt, sowie eine zweite Fraktion, die die Polyoxymethylendimethylether der Formeln H₃C-O-(CH₂O)₃₋₅-CH₃ und H₃C-O-(CH₂O)ₙ-CH₃ mit n≥6 enthält und die Destillationseinheit D1 als Sumpfstrom SS_{D1} verlässt,
- Mischen des Kopfstroms KS_{D1} mit einem methanolhaltigen Strom S_{MeOH} unter Erhalt eines Gemisches M1,
- Umsetzen des Gemisches M1 in mindestens einer Reaktionszone RZ einer Reaktivdestillationseinheit RD2 in Gegenwart eines sauren Katalysators, wobei H₃C-O-(CH₂O)₂-CH₃ zu Methylal und Formaldehyd reagiert und Formaldehyd und Methanol zu Methylal reagieren; und destillatives Auftrennen in eine erste Fraktion, die Methylal (OME₁) enthält und die Reaktivdestillations-einheit RD2 als Kopfstrom KS_{RD2} verlässt, und eine zweite Fraktion, die Wasser enthält und die Reaktivdestillationseinheit RD2 als Sumpfstrom SS_{RD2} verlässt,
- Einleiten des Sumpfstroms SS_{D1} in eine Destillationseinheit D3 und destillatives Auftrennen der im Sumpfstrom SS_{D1} vorliegenden Polyoxymethylendimethylether der Formeln H₃C-O-(CH₂O)₃₋₅-CH₃ und H₃C-O-(CH₂O)ₙ-CH₃ mit n≥6 in eine Fraktion, die die Destillationseinheit D3 als Kopfstrom KS_{D3} verlässt, und eine Fraktion, die die Destillationseinheit als Sumpfstrom SS_{D3} verlässt.

2. Verfahren zur Herstellung von Polyoxymethylendimethylethern, folgende Schritte umfassend:
- Mischen eines Stroms S_{FA}, der eine wasserhaltige Formaldehydquelle enthält, mit einem Strom S_{OME1}, der Methylal enthält, unter Erhalt eines Reaktantengemisches M_{Reaktant},
- Umsetzen des Reaktantengemisches M_{Reaktant} in einem Reaktor R1 unter Erhalt eines Produktgemisches M_{R1}, das Polyoxymethylendimethylether der Formeln H₃C-O-(CH₂O)₂-CH₃, H₃C-O-(CH₂O)₃₋₅-CH₃ und H₃C-O-(CH₂O)ₙ-CH₃ mit n≥6 sowie Formaldehyd, Methylal, Methanol und Wasser enthält,
- Mischen des Produktgemisches M_{R1} mit einem methanolhaltigen Strom S_{MeOH} unter Erhalt eines Gemisches M1,
- Umsetzen des Gemisches M1 in mindestens einer Reaktionszone RZ einer Reaktivdestillationseinheit RD1 in Gegenwart eines sauren Katalysators, wobei H₃C-O-(CH₂O)₂-CH₃ zu Methylal und Formaldehyd reagiert und Formaldehyd und Methanol zu Methylal reagieren; und destillatives Auftrennen in eine erste Fraktion, die Wasser, Methanol, Formaldehyd, Methylal und
H₃C-O-(CH₂O)₂-CH₃ (OME₂) enthält und die Reaktivdestillationseinheit RD1 als Kopfstrom KS_{RD1} verlässt, und eine zweite Fraktion, die die Polyoxymethylendimethylether der Formeln H₃C-O-(CH₂O)₃₋₅-CH₃ und H₃C-O-(CH₂O)ₙ-CH₃ mit n≥6 enthält und die Reaktivdestillationseinheit RD1 als Sumpfstrom SS_{RD1} verlässt,
- Einleiten des Kopfstroms KS_{RD1} in eine Destillationseinheit D2 und destillatives Auftrennen in eine erste Fraktion, die Methylal enthält und die Destillationseinheit D2 als Kopfstrom KS_{D2} verlässt, und eine zweite Fraktion, die Wasser enthält und die Destillationseinheit D2 als Sumpfstrom SS_{D2} verlässt,
- Einleiten des Sumpfstroms SS_{RD1} in eine Destillationseinheit D3 und destillatives Auftrennen der im Sumpfstrom SS_{RD1} vorliegenden Polyoxymethylendimethylether der Formeln H₃C-O-(CH₂O)₃₋₅-CH₃ und H₃C-O-(CH₂O)ₙ-CH₃ mit n≥6 in eine Fraktion, die die Destillationseinheit D3 als Kopfstrom KS_{D3} verlässt, und eine Fraktion, die die Destillationseinheit als Sumpfstrom SS_{D3} verlässt.

3. Verfahren nach Anspruch 1 oder 2, wobei die wasserhaltige Formaldehydquelle eine wässrige Formaldehydlösung mit einem Formaldehydgehalt von mindestens 70 Gew% ist.

4. Verfahren nach einem der vorstehenden Ansprüche, wobei die Umsetzung des Reaktantengemisches M_{Reaktant} in dem Reaktor R1 in Anwesenheit eines sauren Katalysators erfolgt.

5. Verfahren nach einem der vorstehenden Ansprüche, wobei zumindest ein Teil des aus der Reaktivdestillationseinheit RD2 abgezogenen Kopfstroms KS_{RD2} oder des aus der Destillationseinheit D2 abgezogenen Kopfstroms KS_{D2} zurückgeführt wird und als methylalhaltiger Strom S_{OME1} fungiert, der mit dem formaldehyd- und wasserhaltigen Strom S_{FA} unter Erhalt des Reaktantengemisches M_{Reaktant} gemischt wird.

6. Verfahren nach Anspruch 5, wobei der Kopfstrom KS_{RD2} oder KS_{D2} bei seiner Rückführung einen Massenstromteiler passiert, in dem ein Teil des Kopfstroms KS_{RD2} oder KS_{D2} abgezweigt wird.

7. Verfahren nach einem der vorstehenden Ansprüche, wobei der aus der Reaktivdestillationseinheit RD2 abgezogene Sumpfstrom SS_{RD2} oder der aus der Destillationseinheit D2 abgezogene Sumpfstrom SS_{D2} neben Wasser noch Methanol in einem Anteil in einem Anteil von bis zu 80 Gew% enthält, wobei in Summe der Anteil von Wasser und Methanol im Sumpfstrom SS_{RD2} mehr als 95 Gew% beträgt.

8. Verfahren nach einem der Ansprüche 1-6, wobei der aus der Reaktivdestillationseinheit RD2 abgezogene Sumpfstrom SS_{RD2} oder der aus der Destillationseinheit D2 abgezogene Sumpfstrom SS_{D2} neben Wasser noch Formaldehyd in einem Anteil von bis zu 60 Gew% enthält und in Summe der Anteil von Wasser und Formaldehyd im Sumpfstrom SS_{RD2} mehr als 80 Gew%, bevorzugter mehr als 95 Gew% beträgt.

9. Verfahren nach Anspruch 8, wobei der aus der Reaktivdestillationseinheit RD2 abgezogene Sumpfstrom SS_{RD2} oder der aus der Destillationseinheit D2 abgezogene Sumpfstrom SS_{D2} in eine Konzentratoreinheit FC zurückgeführt wird, wobei in der Konzentratoreinheit FC ein Teil des Wassers entfernt wird und ein Strom die Konzentratoreinheit FC verlässt, der als Strom S_{FA} fungiert und mit dem methylalhaltigen Strom S_{OME1} unter Erhalt des Reaktantengemisches M_{Reaktant} gemischt wird.

10. Verfahren nach Anspruch 9, wobei der zurückgeführte formaldehydhaltige Sumpfstrom SS_{RD2} oder SS_{D2} vor dem Einleiten in die Konzentratoreinheit FC mit einem formaldehydhaltigen Ausgangsmaterial, insbesondere einer wässrigen Formaldehydlösung, unter Erhalt eines formaldehydhaltigen Gemisches M2 gemischt und das formaldehydhaltige Gemisch M2 in die Konzentratoreinheit FC eingeleitet wird.

11. Verfahren nach Anspruch 9 oder 10, wobei die Konzentratoreinheit FC mindestens einen Verdampfer, insbesondere mindestens einen Dünnschichtverdampfer, enthält.

12. Verfahren nach einem der vorstehenden Ansprüche, wobei der aus der Destillationseinheit D3 abgezogene Kopfstrom KS_{D3} H₃C-O-(CH₂O)₃₋₅-CH₃ enthält und der Sumpfstrom SS_{D3} Polyoxymethylendimethylether der Formel H₃C-O-(CH₂O)ₙ-CH₃ mit n≥6 enthält.

13. Verfahren nach einem der vorstehenden Ansprüche, wobei der Sumpfstrom SS_{D3} zurückgeführt und mit dem Strom S_{FA} und/oder dem Strom S_{OME1} gemischt wird.

## Claims

1. A process for preparing polyoxymethylene dimethyl ethers, comprising the following steps:
- mixing of a stream S_{FA} containing a water-containing formaldehyde source with a stream S_{OME1} containing methylal, to obtain a reactant mixture M_{Reactant},
- reaction of the reactant mixture M_{Reactant} in a reactor R1 to obtain a product mixture M_{R1} containing polyoxymethylene dimethyl ethers of the formulae H₃C-O-(CH₂O)₂-CH₃, H₃C-O-(CH₂O)₃₋₅-CH₃ and H₃C-O-(CH₂O)ₙ-CH₃ with n ≥ 6 and also formaldehyde, methylal, methanol and water,
- introduction of the product mixture M_{R1} into a distillation unit D1 and distillative separation of the product mixture M_{R1} into a first fraction which contains methylal, H₃C-O-(CH₂O)₂-CH₃, formaldehyde, methanol and water and leaves the distillation unit D1 as top stream KS_{D1}, and a second fraction which contains the polyoxymethylene dimethyl ethers of the formulae H₃C-O-(CH₂O)₃₋₅-CH₃ and H₃C-O-(CH₂O)ₙ-CH₃ with n ≥ 6 and leaves the distillation unit D1 as bottom stream SS_{D1},
- mixing of the top stream KS_{D1} with a methanol-containing stream S_{MeOH} to obtain a mixture M1,
- reaction of the mixture M1 in at least one reaction zone RZ of a reactive distillation unit RD2 in the presence of an acid catalyst, wherein H₃C-O-(CH₂O)₂-CH₃ reacts to give methylal and formaldehyde, and formaldehyde and methanol react to give methylal; and distillative separation into a first fraction which contains methylal (OME₁) and leaves the reactive distillation unit RD2 as top stream KS_{RD2}, and a second fraction which contains water and leaves the reactive distillation unit RD2 as bottom stream SS_{RD2},
- introduction of the bottom stream SS_{D1} into a distillation unit D3 and distillative separation of the polyoxymethylene dimethyl ethers of the formulae H₃C-O-(CH₂O)₃₋₅-CH₃ and
H₃C-O-(CH₂O)ₙ-CH₃ with n ≥ 6 present in the bottom stream SS_{D1} into a fraction which leaves the distillation unit D3 as top stream KS_{D3}, and a fraction which leaves the distillation unit as bottom stream SS_{D3}.

2. A process for preparing polyoxymethylene dimethyl ethers, comprising the following steps:
- mixing of a stream S_{FA} containing a water-containing formaldehyde source with a stream S_{OME1} containing methylal, to obtain a reactant mixture M_{Reactant},
- reaction of the reactant mixture M_{Reactant} in a reactor R1 to obtain a product mixture M_{R1} containing polyoxymethylene dimethyl ethers of the formulae H₃C-O-(CH₂O)₂-CH₃, H₃C-O-(CH₂O)₃₋₅-CH₃ and
H₃C-O-(CH₂O)ₙ-CH₃ with n ≥ 6 and also formaldehyde, methylal, methanol and water,
- mixing of the product mixture M_{R1} with a methanol-containing stream S_{MeOH} to obtain a mixture M1,
- reaction of the mixture M1 in at least one reaction zone RZ of a reactive distillation unit RD1 in the presence of an acid catalyst, wherein H₃C-O-(CH₂O)₂-CH₃ reacts to give methylal and formaldehyde, and formaldehyde and methanol react to give methylal; and distillative separation into a first fraction which contains water, methanol, formaldehyde, methylal and H₃C-O-(CH₂O)₂-CH₃ (OME₂) and leaves the reactive distillation unit RD1 as top stream KS_{RD1}, and a second fraction which contains the polyoxymethylene dimethyl ethers of the formulae H₃C-O-(CH₂O)₃₋₅-CH₃ and H₃C-O-(CH₂O)ₙ-CH₃ with n ≥ 6 and leaves the reactive distillation unit RD1 as bottom stream SS_{RD1},
- introduction of the top stream KS_{RD1} into a distillation unit D2 and distillative separation into a first fraction which contains methylal and leaves the distillation unit D2 as top stream KS_{D2}, and a second fraction which contains water and leaves the distillation unit D2 as bottom stream SS_{D2},
- introduction of the bottom stream SS_{RD1} into a distillation unit D3 and distillative separation of the polyoxymethylene dimethyl ethers of formulae H₃C-O-(CH₂O)₃₋₅-CH₃ and H₃C-O-(CH₂O)ₙ-CH₃ with n ≥ 6 present in the bottom stream SS_{RD1} into a fraction which leaves the distillation unit D3 as top stream KS_{D3}, and a fraction which leaves the distillation unit as bottom stream SS_{D3}.

3. The process as claimed in claim 1 or 2, wherein the water-containing formaldehyde source is an aqueous formaldehyde solution having a formaldehyde content of at least 70% by weight.

4. The process as claimed in any of the preceding claims, wherein the reactant mixture M_{Reactant} is reacted in the reactor R1 in the presence of an acidic catalyst.

5. The process as claimed in any of the preceding claims, wherein at least a portion of the top stream KS_{RD2} drawn off from the reactive distillation unit RD2 or of the top stream KS_{D2} drawn off from the distillation unit D2 is recycled and functions as methylal-containing stream S_{OME1}, which is mixed with the formaldehyde- and water-containing stream S_{FA} to obtain the reactant mixture M_{Reactant}.

6. The process as claimed in claim 5, wherein the top stream KS_{RD2} or KS_{D2} during the recycling thereof passes through a mass flow divider in which a portion of the top stream KS_{RD2} or KS_{D2} is branched off.

7. The process as claimed in any of the preceding claims, wherein the bottom stream SS_{RD2} drawn off from the reactive distillation unit RD2 or the bottom stream SS_{D2} drawn off from the distillation unit D2 also contains, besides water, methanol in a proportion of up to 80% by weight, wherein the total proportion of water and methanol in the bottom stream SS_{RD2} is more than 95% by weight.

8. The process as claimed in any of claims 1-6, wherein the bottom stream SS_{RD2} drawn off from the reactive distillation unit RD2 or the bottom stream SS_{D2} drawn off from the distillation unit D2 also contains, besides water, formaldehyde in a proportion of up to 60% by weight and the total proportion of water and formaldehyde in the bottom stream SS_{RD2} is more than 80% by weight, more preferably more than 95% by weight.

9. The process as claimed in claim 8, wherein the bottom stream SS_{RD2} drawn off from the reactive distillation unit RD2 or the bottom stream SS_{D2} drawn off from the distillation unit D2 is recycled into a concentrator unit FC, wherein in the concentrator unit FC a portion of the water is removed and a stream leaves the concentrator unit FC which functions as stream S_{FA} and is mixed with the methylal-containing stream S_{OME1} to obtain the reactant mixture M_{Reactant}.

10. The process as claimed in claim 9, wherein the recycled formaldehyde-containing bottom stream SS_{RD2} or SS_{D2} prior to being introduced into the concentrator unit FC is mixed with a formaldehyde-containing starting material, in particular an aqueous formaldehyde solution, to obtain a formaldehyde-containing mixture M2, and the formaldehyde-containing mixture M2 is introduced into the concentrator unit FC.

11. The process as claimed in claim 9 or 10, wherein the concentrator unit FC comprises at least one evaporator, in particular at least one thin-film evaporator.

12. The process as claimed in any of the preceding claims, wherein the top stream KS_{D3} drawn off from the distillation unit D3 contains H₃C-O-(CH₂O)₃₋₅-CH₃ and the bottom stream SS_{D3} contains polyoxymethylene dimethyl ethers of the formula H₃C-O-(CH₂O)ₙ-CH₃ with n ≥ 6.

13. The process as claimed in any of the preceding claims, wherein the bottom stream SS_{D3} is recycled and mixed with the stream S_{FA} and/or the stream S_{OME1}.

## Revendications

1. Procédé de préparation de polyoxyméthylènediméthyléthers, comprenant les étapes suivantes :
- mélange d'un courant S_{FA} qui contient une source aqueuse de formaldéhyde, avec un courant S_{OME1} qui contient du méthylal, avec obtention d'un mélange de réactifs M_{Réactif},
- réaction du mélange de réactifs M_{Réactif} dans un réacteur R1 avec obtention d'un mélange de produits M_{R1} qui contient des polyoxyméthylènediméthyléthers de formules H₃C-O-(CH₂O)₂-CH₃, H₃C-O-(CH₂O)₃₋₅-CH₃ et H₃C-O-(CH₂O)ₙ-CH₃ avec n ≥ 6, ainsi que du formaldéhyde, du méthylal, du méthanol et de l'eau,
- introduction du mélange de produits M_{R1} dans une unité de distillation D1 et séparation par distillation du mélange de produits M_{R1} en une première fraction qui contient du méthylal, H₃C-O-(CH₂O)₂-CH₃, du formaldéhyde, du méthanol et de l'eau, et quitte l'unité de distillation D1 en tant que courant de tête KS_{D1}, ainsi qu'une deuxième fraction qui contient les polyoxyméthylènediméthyléthers de formules H₃C-O-(CH₂O)₃₋₅-CH₃ et H₃C-O-(CH₂O)ₙ-CH₃ avec n ≥ 6 et quitte l'unité de distillation D1 en tant que courant de fond SS_{D1},
- mélange du courant de tête KS_{D1} avec un courant S_{MeOH} contenant du méthanol, avec obtention d'un mélange M1,
- réaction du mélange M1 dans au moins une zone de réaction RZ d'une unité de distillation réactive RD2 en présence d'un catalyseur acide, le H₃C-O-(CH₂O)₂-CH₃ réagissant pour donner du méthylal et du formaldéhyde, et le formaldéhyde et le méthanol réagissant pour donner du méthylal, et séparation par distillation en une première fraction qui contient du méthylal (OME₂) et quitte l'unité de distillation réactive RD2 en tant que courant de tête KS_{RD2}, et une deuxième fraction qui contient de l'eau et qui quitte l'unité de distillation réactive RD2 en tant que courant de fond SS_{RD2},
- introduction du courant de fond SS_{D1} dans une unité de distillation D3 et séparation par distillation des polyoxyméthylènediméthyléthers présents dans le courant de fond SS_{D1} de formules H₃C-O-(CH₂O)₃₋₅-CH₃ et H₃C-O-(CH₂O)ₙ-CH₃ avec n ≥ 6 en une fraction qui quitte l'unité de distillation D3 en tant que courant de tête KS_{D3} et une fraction qui quitte l'unité de distillation en tant que courant de fond SS_{D3}.

2. Procédé de préparation de polyoxyméthylènediméthyléthers, comprenant les étapes suivantes :
- mélange d'un courant S_{FA} qui contient une source aqueuse de formaldéhyde avec un courant S_{OME1} qui contient du méthylal, avec obtention d'un mélange de réactifs M_{Réactif},
- réaction du mélange de réactifs M_{Réactif} dans un réacteur R1 avec obtention d'un mélange de produits M_{R1} qui contient des polyoxyméthylènediméthyléthers de formules H₃C-O-(CH₂O)₂-CH₃, H₃C-O-(CH₂O) ₃₋₅-CH₃ et H₃C-O-(CH₂O)ₙ-CH₃ avec n ≥ 6, ainsi que du formaldéhyde, du méthylal, du méthanol et de l'eau,
- mélange du mélange de produits M_{R1} avec un courant S_{MeOH} contenant du méthanol, avec obtention d'un mélange M1,
- réaction du mélange M1 dans au moins une zone de réaction RZ d'une unité de distillation réactive RD1 en présence d'un catalyseur acide, le H₃C-O-(CH₂O)₂-CH₃ réagissant pour donner du méthylal et du formaldéhyde, et le formaldéhyde et le méthanol réagissant pour donner du méthylal ; et séparation par distillation en une première fraction qui contient de l'eau, du méthanol, du formaldéhyde, du méthylal et du H₃C-O-(CH₂O)₂-CH₃(OME₂) et quitte l'unité de distillation réactive RD1 en tant que courant de tête KS_{RD1} et une deuxième fraction qui contient les polyoxyméthylènediméthyléthers de formules H₃C-O-(CH₂O)₃₋₅-CH₃ et H₃C-O-(CH₂O)ₙ-CH₃ avec n ≥ 6, et quitte l'unité de distillation réactive RD1 en tant que courant de fond SS_{RD1},
- introduction du courant de tête KS_{RD1} dans une unité de distillation D2 et séparation par distillation en une première fraction qui contient du méthylal et quitte l'unité de distillation D2 en tant que courant de tête KS_{D2} et une deuxième fraction qui contient de l'eau et quitte l'unité de distillation D2 en tant que courant de fond SS_{D2},
- introduction du courant de fond SS_{RD1} dans une unité de distillation D3 et séparation par distillation des polyoxyméthylènediméthyléthers, présents dans le courant de fond SS_{RD1}, de formules H₃C-O-(CH₂O)₃₋₅-CH₃ et H₃C-O-(CH₂O)ₙ-CH₃ avec n ≥ 6 en une fraction qui quitte l'unité de distillation D3 en tant que courant de tête KS_{D3} et une fraction qui quitte l'unité de distillation en tant que courant de fond SS_{D3}.

3. Procédé selon la revendication 1 ou 2, dans lequel la source aqueuse de formaldéhyde est une solution aqueuse de formaldéhyde ayant une teneur en formaldéhyde d'au moins 70 % en poids.

4. Procédé selon l'une des revendications précédentes, dans lequel la réaction du mélange de réactifs M_{Réactif} dans le réacteur R1 a lieu en présence d'un catalyseur acide.

5. Procédé selon l'une des revendications précédentes, dans lequel au moins une partie du courant de tête KS_{RD2} soutiré de l'unité de distillation réactive RD2 ou du courant de tête KS_{D2} soutiré de l'unité de distillation D2 est renvoyée en reflux et joue le rôle du courant S_{OME1} contenant du méthylal, qui est mélangé avec le courant S_{FA} contenant du formaldéhyde et de l'eau, avec obtention du mélange de réactifs M_{Réactif}.

6. Procédé selon la revendication 5, dans lequel le courant de tête KS_{RD2} ou KS_{D2} passe, lors de son retour, par un diviseur de débit massique, dans lequel est soutirée une partie du courant de tête KS_{RD2} ou KS_{D2}.

7. Procédé selon l'une des revendications précédentes, dans lequel le courant de fond SS_{RD2} soutiré de l'unité de distillation réactive RD2 ou le courant de fond SS_{D2} soutiré de l'unité de distillation D2 contient encore, outre de l'eau, du méthanol selon une proportion allant jusqu'à 80 % en poids, la proportion totale de l'eau et du méthanol dans le courant de fond SS_{RD2} étant supérieure à 95 % en poids.

8. Procédé selon l'une des revendications 1 à 6, dans lequel le courant de fond SS_{RD2} soutiré de l'unité de distillation réactive RD2 ou le courant de fond SS_{D2} soutiré de l'unité de distillation D2 contient encore, outre de l'eau, du formaldéhyde selon une proportion allant jusqu'à 60 % en poids, et la proportion totale de l'eau et du formaldéhyde dans le courant de fond SS_{RD2} étant supérieure à 80 % en poids, plus préférentiellement supérieure à 95 % en poids.

9. Procédé selon la revendication 8, dans lequel le courant de fond SS_{RD2} soutiré de l'unité de distillation réactive RD2 ou le courant de fond SS_{D2} soutiré de l'unité de distillation D2 est renvoyé dans une unité de concentration FC, une partie de l'eau étant éliminée dans l'unité de concentration FC, et un courant quittant l'unité de concentration FC, qui joue le rôle de courant S_{FA} et est mélangé avec le courant S_{OME1} contenant du méthylal avec obtention du mélange de réactifs M_{Réactif}.

10. Procédé selon la revendication 9, dans lequel le courant de fond SS_{RD2} ou SS_{D2} contenant du formaldéhyde est renvoyé et, avant introduction dans l'unité de concentration FC, mélangé avec un matériau de départ contenant du formaldéhyde, en particulier une solution aqueuse de formaldéhyde, avec obtention d'un mélange M2 contenant du formaldéhyde, et le mélange M2 contenant du formaldéhyde est introduit dans l'unité de concentration FC.

11. Procédé selon la revendication 9 ou 10, dans lequel l'unité de concentration FC contient au moins un évaporateur, en particulier au moins un évaporateur à couche mince.

12. Procédé selon l'une des revendications précédentes, dans lequel le courant de tête KS_{D3} soutiré de l'unité de distillation D3 contient du H₃C-O-(CH₂O)₃₋₅-CH₃ et le courant de fond SS_{D3} contient des polyoxyméthylènediméthyléthers de formule H₃C-O-(CH₂O)ₙ-CH₃ avec n ≥ 6.

13. Procédé selon l'une des revendications précédentes, dans lequel le courant de fond SS_{D3} est renvoyé et mélangé au courant S_{FA} et/ou au courant S_{OME1}.
